(19) Europäisches Patentamt
European Patent Office
Office européen des brevets

(11) EP 4 435 021 A1

(12) EUROPEAN PATENT APPLICATION
published in accordance with Art. 153(4) EPC

(43) Date of publication:
**25.09.2024 Bulletin 2024/39**

(21) Application number: **22895583.7**

(22) Date of filing: **14.11.2022**

(51) International Patent Classification (IPC):
***C08F 220/36*** (2006.01) ***C08F 290/06*** (2006.01)
***C09D 4/02*** (2006.01) ***C09D 5/00*** (2006.01)
***C09D 11/101*** (2014.01) ***C09D 11/30*** (2014.01)
***C09J 4/02*** (2006.01)

(52) Cooperative Patent Classification (CPC):
**C08F 220/36; C08F 290/06; C09D 4/00; C09D 5/00; C09D 11/101; C09D 11/30; C09J 4/00**

(86) International application number:
**PCT/JP2022/042300**

(87) International publication number:
**WO 2023/090302 (25.05.2023 Gazette 2023/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.11.2021 JP 2021187434**
**17.11.2021 JP 2021187438**

(71) Applicant: **KJ Chemicals Corporation**
**Tokyo 103-0023 (JP)**

(72) Inventors:
- **HIRATA Meiri**
**Tokyo 103-0023 (JP)**
- **YASUNAGA Atsushi**
**Yatsushiro-shi Kumamoto 866-0081 (JP)**
- **DAS Asmit**
**Yatsushiro-shi Kumamoto 866-0081 (JP)**

(74) Representative: **ABG Intellectual Property Law, S.L.**
**Avenida de Burgos, 16D**
**Edificio Euromor**
**28036 Madrid (ES)**

# (54) ACTIVE ENERGY RAY-CURABLE COMPOSITION

(57) [Problem] The present invention addresses the problem of providing: an active energy ray curable composition which has various characteristics required for active energy ray curable compositions, and which is especially capable of achieving high transparency, high curability with respect to LED rays, and resistance to curing shrinkage at the same time, the characteristics having been difficult to achieve at the same time; and a cured product which can achieve transparency, heat resistance, impact resistance, wear resistance, resistance to wet heat yellowing, water resistance and durability at the same time, the characteristics having been difficult to achieve at the same time. [Solution] The present invention provides an active energy ray curable composition which contains a (meth)acrylate (A) that has one or more amide groups and one or more cyclic substituents in each molecule, and a polymerizable compound (B) (excluding (A)) that has one or more ethylenically unsaturated groups in each molecule, and which contains, as the polymerizable compound (B), at least a polymerizable compound (b 1) that has one or more chain substituents having 1 to 36 carbon atoms and/or a polymerizable compound (b2) that has one or more cyclic substituents having 3 to 20 carbon atoms.

EP 4 435 021 A1

## Description

TECHNICAL FIELD

The present invention relates to an active-energy ray curable composition, curable ink composition, pressure-sensitive adhesive composition, adhesive composition, sealing composition, coating composition, nail cosmetic, decorative coating agent, dental material, and cured product obtained by curing thereof.

BACKGROUD

Active energy ray curable compositions are generally composed of multiple polymerizable components and a photopolymerization initiator, and can be solidified (cured) from liquid state at room temperature in a short time by irradiation with active energy rays such as ultraviolet rays (UV) or electron beams (EB), and are used in a wide range of fields such as coating agents, paints, pressure-sensitive adhesives, adhesives, elastomer-based materials, materials for flexible displays, inkjet inks, sealing materials, sealants, dental hygiene materials, and optical materials. In particular, they are widely used as nail cosmetics such as gel nails and as materials for two dimensional or three-dimensional modeling because they can be cured in any place or shape.

Active energy ray curable compositions are required to have various performances depending on the fields and environments in which they are used, and it is necessary to combine various kinds of polymerizable components in order to cope with the requirements. In general, a polymerizable oligomer (prepolymer) such as urethane acrylate or epoxy acrylate, a polyfunctional monomer, a monofunctional monomer, and a photopolymerization initiator are blended as main components, and additives such as fillers, pigments or dyes, leveling agents, viscosity modifiers, and polymerization inhibitors can be added depending on the use. For example, Patent Literature 1 describes an active energy ray curable composition containing monofunctional monomers such as (meth)acrylate having a cycloaliphatic skeleton, N-substituted (meth)acrylamide, (meth)acrylate having a linear or branched alkyl having 8 to 18 carbon atoms, polymerizable oligomers such as (meth)acrylate having urethane groups, and a photopolymerization initiator. It has been proposed to provide a cured product having both high elongation and high elastic modulus at a high level by blending these components in a specific proportion. The patent document discloses that a monofunctional (meth)acrylate having a cycloaliphatic skeleton and a monofunctional (meth)acrylate having a linear or branched alkyl having 8 to 18 carbon atoms are preferable from the viewpoint of elongation and modulus of the cured product, a N-substituted (meth)acrylamide having an extremely short strand alkyl such as dimethyl or diethyl is preferable from the viewpoint of curability, and a urethane (meth)acrylate having 1 to 2 (meth)acrylic group(s) is preferable from the viewpoint of elongation of the cured product, but does not describe transparency of the curable composition and transparency, heat resistance, impact resistance, durability, water resistance, and the like of the obtained cured product. It is known that both the (meth)acrylate having a cycloaliphatic skeleton and the (meth)acrylate having a linear or branched alkyl having 8 to 18 carbon atoms are highly hydrophobic and have high steric hindrance, so that curability is low, whereas N-substituted (meth)acrylamide having short strand alkyl has high hydrophilicity and curability. With such a combination of components, a curable composition and a cured product thereof cannot be expected to exhibit good transparency, and there has been a problem that it is difficult to use them in the optical field such as an optical member. In addition, due to the difference in curability, the molecular level structure of the obtained cured product becomes non-uniform, shrinkage at the time of curing is large, and internal stress and strain of the obtained cured product easily occur, and there is a problem that sufficient durability, impact resistance, and heat resistance as a paint, a pressure-sensitive adhesive, an adhesive, an electronic material, a dental material, a three-dimensional modeling object, or the like cannot be provided.

Patent Literature 2 describes an active energy ray curable composition containing a multicyclic carboxamide having a (meth)acryloyl. In addition, the curable resin composition described in this literature had good UV curability, and the obtained cured film had good surface hardness as a hard coat.

CITATION LIST

Patent Literature

Patent Literature 1: JP 2021-123720 A
Patent Literature 2: JP 2018-95626 A

## SUMMARY OF THE INVENTION

### Technical Problem

An object of the present invention is to provide a novel active energy ray curable composition having a wide variety of properties required for an active energy ray curable composition, and particularly having high transparency, high curability with respect to LED light, and resistance to curing shrinkage, which have been difficult to achieve in parallel.

Another object of the present invention is to provide a cured product having a wide variety of properties required for a cured product of an active energy ray curable composition, and particularly having transparency, heat resistance, impact resistance, water resistance, durability, and impact resistance which have been difficult to achieve in parallel.

### Solution to Problem

As a result of intensive studies, the present inventors have found that an active energy ray curable composition containing a (meth)acrylate (A) having one or more amide groups and one or more cyclic substituents in the molecule, and a polymerizable compound (B) (excluding A) having one or more ethylenically unsaturated groups in the molecule. The polymerizable compound (B) includes at least a polymerizable compound (b1) having one or more chain substituents having 1 to 36 carbon atoms and/or a polymerizable compound (b2) having one or more cyclic substituents having 3 to 20 carbon atoms. The active energy ray curable composition can solve the problems mentioned above, thereby completing the present invention.

That is, the present invention is based on the following constitution.

(1) An active energy ray curable composition comprising a (meth)acrylate (A) having one or more amide groups and one or more cyclic substitutions in the molecule, and a polymerizable compound (B) (excluding A) having one or more ethylenically unsaturated groups in the molecule, wherein the polymerizable compound (B) at least contains a polymerizable compound (b1) having one or more chain substituents having 1 to 36 carbon atoms and/or a polymerizable compound (b2) having one or more cyclic substituents having 3 to 20 carbon atoms.
(2) The active energy ray curable composition according to (1), wherein the substituents of the (meth)acrylate (A) are one or more substituents selected from represents saturated or unsaturated multicyclic aliphatic rings, mono- or multicyclic aromatic rings, saturated or unsaturated aliphatic heterocycles, and aromatic heterocycles.
(3) The active energy ray curable composition according to (1) or (2), wherein the (meth)acrylate (A) has an amide group between the (meth)acrylate and the cyclic substituents.
(4) The active energy ray curable composition according to any one of (1) to (3), wherein the polymerizable compound (B) has one or more groups selected from (meth)acrylamide, (meth)acrylate, vinyl groups, vinyl ether, alkyl vinyl ether, allyl groups, (meth)allyl ether, and maleimide as the ethylenically unsaturated group.
(5) The active energy ray curable composition according to any one of (1) to (4), wherein the content of (meth)acrylate (A) with respect to the entire active energy ray curable composition is 0. 1 to 99.5% by mass, the content of the polymerizable compound (b1) is 0.3 to 80% by mass, and the content of the polymerizable compound (b2) is 0.2 to 70% by mass.
(6) The active energy ray curable composition according to any one of (1) to (5), which has a curing shrinkage rate of 5% or less.
(7) The active energy ray curable composition according to any one of (1) to (6), wherein its cured product has a saturated water absorption rate of 10% or less.
(8) An ink composition comprising the active energy ray curable composition according to any one of (1) to (7).
(9) An ink composition for two-dimensional or three-dimensional modeling comprising the active energy ray curable composition according to any one of (1) to (7).
(10) A pressure-sensitive adhesive composition comprising the active energy ray curable composition according to any one of (1) to (7).
(11) An adhesive composition comprising the active energy ray curable composition according to any one of (1) to (7).
(12) A coating composition comprising the active energy ray curable composition according to any one of (1) to (7).
(13) A sealing composition comprising the active energy ray curable composition according to any one of (1) to (7).
(14) A nail cosmetic comprising the active energy ray curable composition according to any one of (1) to (7).
(15) A decorative coating agent comprising the active energy ray curable composition according to any one of (1) to (7).
(16) A dental comprising the active energy ray curable composition according to any one of (1) to (7).

Advantageous Effects of Invention

According to the present invention, an active energy ray curable composition containing a (meth)acrylate (A) having a specific structure and a polymerizable compound (B) excluding A has high transparency and curability, since A has well-balanced amphiphilicity and high curability, so that A exhibits good compatibility with B of various types from hydrophobic to hydrophilic and from monofunctional to polyfunctional. In addition, by having one or more cyclic substituents in the molecule of the (meth)acrylate (A) and by containing a polymerizable compound (b1) having one or more chain substituents having 1 to 36 carbon atoms as the polymerizable compound (B), the chain substituents derived from b1 were easily filled and arranged between and around the cyclic substituents derived from A, and the volume shrinkage in the curing process of the active energy ray curable composition could be suppressed to a low level. Furthermore, by containing the polymerizable compound (b1) and the polymerizable compound (b2) at the same time, the steric hindrance between the (meth)acrylate (A) and the cyclic substituents of b2 are eliminated by the chain substituents of b1. The active energy ray curable composition containing A, b1, and b2 has high curability, low curing shrinkage, tends to maintain a good compatibility state, and the resulting cured product has good transparency, heat resistance and durability.

(Meth)acrylate (A) has cyclic substituents in the molecules and has a high index of refraction. Due to the interaction between the cyclic substituents and the intramolecular amide groups, the (meth)acrylate (A) homopolymer has a higher glass-transition temperature (Tg) than ordinary cyclic polymers. In the cured product obtained from the containing (meth)acrylate(A) having such a specific structure and a polymerizable compound (B) active energy ray curable composition, high transparency, heat resistance, durability and impact resistance were realized. Further, the cured product obtained from the active energy ray curable composition of the present invention has high resistance to yellowing and high-water resistance while having an amide group which is generally recognized as being easily colored and having low water resistance in as an advantageous effect of the interaction between the amide groups and the cyclic substituents of (meth)acrylate (A). When (meth)acrylate (A) has an unsaturated multicyclic aliphatic ring and/or an unsaturated aliphatic heterocyclic ring, the unsaturated bonds of the rings are partially polymerized by irradiation with active energy rays, so that A becomes a special monomer between monofunctional and polyfunctional, and the resulting cured product is excellent in impact resistance.

The active energy ray curable composition of the present invention has high curability with respect to light (wave length range: 250 to 450 nm) from a general-purpose metal halide lamp for curing UV inks and with respect to high-safety LED light (wave length 365 nm, 385 nm, 395 nm, 405 nm), and the liquid viscosity of the curable composition can be adjusted from low viscosity to high viscosity almost without limitation depending on the application, and can be provided as a curable ink composition for printing such as inkjet printing, screen printing, offset printing, and flexo printing, and as a curable ink composition for two-dimensional or three-dimensional modeling. Further, the active energy ray curable composition of the present invention has high wettability and adhesion to various substrates ranging from low-polarity plastics to high-polarity glasses and metals, and can be provided as a curable pressure-sensitive adhesive composition, an adhesive composition, a coating composition, sealing composition, a nail cosmetic, decorative coating agent for decorative film or decorative sheet, dental materials and the like.

Description of Embodiments

Hereinafter, embodiments of the present invention are described in detail.
The first to fifth embodiments of the present invention (hereinafter also collectively referred to as the present embodiment) relate to an active energy ray curable composition (E). The active energy ray curable composition (E) (hereinafter also simply referred to as "curable composition (E)") comprising a (meth)acrylate (A) having one or more amide groups and one or more cyclic substitution groups in the molecule, and a polymerizable compound (B) (excluding A) having one or more ethylenically unsaturated groups in the molecule, and B contains a polymerizable compound (b 1) having a chain substituents having 1 to 36 carbon atoms and/or a polymerizable compound (b2) having one or more cyclic substituents having 3 to 20 carbon atoms.

By containing (meth)acrylate(A), the active energy ray curable composition (E) has high curability, and cure shrinkage when e is cured is suppressed. By the interaction between A and B, the transparency of E is improved, the curing shrinkage of E is further reduced, and the cured product of E has high yellowing resistance and water resistance. Furthermore, since E contains A and b2, a cured product of E has high heat resistance and durability.

With respect to the total mass of the active energy ray curable composition (E), the content of (meth)acrylate (A) is preferably 0.1 to 99.5% by mass, the content of polymerizable compound (b 1) is preferably 0.3 to 80% by mass, and the content of the polymerizable compound (b2) is preferably 0.2 to 70% by mass. The total content of b1 and b2 is more preferably 0.5% by mass or more. When the contents of A, b1, and b2 are within these ranges, the transparency, curability, and curing shrinkage resistance of E can be achieved at the same time, and the resulting cured product has good transparency, heat resistance, yellowing resistance, durability, and water resistance. In addition, in E and its cured product, from the viewpoint of coexisting with the various properties described above while maintaining high performance,

the content of A is more preferably 1 to 95% by mass, the content of b1 is more preferably 5 to 70% by mass, and the content of b2 is more preferably 2 to 60% by mass. The content of A is particularly preferably 5 to 95% by mass, the content of b1 is particularly preferably 10 to 60% by mass, and the content of b2 is particularly preferably 5 to 50% by mass.

The (meth)acrylate (A) having one or more amide groups and one or more cyclic substituents in the molecule is not particularly limited as long as it is a compound having one or more amide groups, one or more cyclic substituents and at one or more methacrylate group and/or acrylate. The cyclic substituents are one or more substituents selected from monocyclic or polycyclic aliphatic rings, monocyclic or polycyclic aromatic rings, aliphatic heterocyclic rings, aromatic heterocyclic rings, and these substituents may be saturated rings or unsaturated rings, and examples include such as cyclohexyl group, dicyclopentanyl group, dicyclopentenyl, bornyl group, isobornyl group, adamantly group, norbornane group, norbornene group, oxanorbornane group, oxanorbornene group, azanorbornane group, azanorbornene group, tricyclodecane group, phenyl group, naphthalene group, triphenylene group, pyrene group, perylene group, indacene group, biphenylene group, acenaphthylene group, phenalene group, anthracene group, cyclohexylphenyl group, 4-morpholino phenyl group, various bisphenol group, bisoxazoline group, and the like. These cyclic substituents may have only one type, or may have two or more types.

The cyclic substituents of (meth)acrylate (A) may further have a substituent (R). The substituent R is one or more groups selected from the group consisting of ethylenically unsaturated bond and optionally substituted carbon linear alkyl groups having a number of 1 to 6 or hydroxyalkylene, alkenyl or alkyleneoxyalkyl having 2 to 6 carbon atoms, a branched alkyl group having 3 to 6 carbon atoms, the ethylenically unsaturated group in R is represented by (meth)acrylate, (meth)acrylamide, vinyl group, vinyl ether, alkyl vinyl ether, allyl group, (meth)allyl ether and maleimide. The cyclic substituents of A may be saturated rings or unsaturated rings. It is preferable that the cyclic substituents of A at least have one unsaturated ring and/or that the substituent R has one ethylenically unsaturated group. In this preferred case, A has two or more unsaturated groups in the molecule, and the curability of the active energy ray curable composition (E) becomes higher, and the heat resistance, durability, water resistance, etc. of the resulting cured product can be further improved. Moreover, it is more preferable that the unsaturated group of the unsaturated ring of A and/or the ethylenically unsaturated group of the substituent R is a group other than a (meth)methacrylate group. In this more preferred case, A has two or more unsaturated groups having different structures in the molecule, and the two or more different unsaturated groups can be cured in order by adjusting the type, intensity, and the like of the active energy ray, and the crosslinking rate of the cured product can be increased stepwise while suppressing the curing shrinkage rate, and a cured product having excellent impact resistance can be obtained. In this more preferred case, A has two or more unsaturated groups with different structures in the molecule, and by adjusting the type and intensity of active energy rays, two or more different unsaturated groups can be cured in sequence, making it possible to gradually increase the crosslinking rate of the cured product while suppressing the curing shrinkage rate, and a cured product having excellent impact resistance can be obtained.

Examples of the amide groups of (meth)acrylate (A) include secondary amide group (N -substituted amide), tertiary amide group (N, N-disubstituted amide), diacetamide, N-substituted diacetamide, and the like. Preferably, A has an amide group between the (meth)acrylate and the cyclic substituents. In this case, the effect of the interaction between the amide group and the (meth)acrylate and/or the interaction between the amide group and the cyclic substituent can be expressed more efficiently than in the case where these groups act alone. In A, the (meth)acrylate and the amide group are preferably linked via a linear or branched alkylene (first linking group) having 1 to 6 carbon atoms, and the amide group and the cyclic substituent are preferably directly linked or linked via a linear or branched alkylene (second linking group) having 1 to 6 carbon atoms. In addition, when the (meth)acrylate and the amide group are linked by a linear alkylene having 2 to 4 carbon atoms, and the amide group and the cyclic substituent are directly linked, the curability of the (meth)acrylate with respect to active energy rays is improved, and the yellowing of the cured product due to the amide group over time is reduced, which is more preferable. Furthermore, in a case where the cyclic substituents are unsaturated rings, curability with respect to an active energy ray is further improved, and thus it is particularly preferable.

The (meth)acrylate (A) can be synthesized by a known method, and generally includes methods such as: carboxylic acid having cyclic substituents, a carboxylic acid ester, a carboxylic acid chloride, a carboxylic acid anhydride, and the like, react with a compound having a hydroxyl group and an amino group such as an alkanolamine by amidation reaction to obtain a carboxamide compound with a hydroxy and cyclic substituents, then the carboxamide compound react with a (meth)acrylic acid anhydride or a (meth)acrylic acid by esterification reaction, or the carboxamide compound react with a lower alkyl (meth)acrylate ester by transesterification reaction; a N-substituted amide or a N,N-disubstituted amide substituted with cyclic substituents having a hydroxy group react with a (meth)acrylic acid anhydride or a (meth)acrylic acid by esterification reaction, or react with a lower alkyl (meth)acrylate ester by transesterification reaction.

Examples of the (meth)acrylate (A) include (meth)acryloyloxyalkylene norbornanecarboxamides such as (meth)acryloyloxymethylene norbornanecarboxyamide, (meth)acryloyloxyethylene norbornanecarboxyamide, (meth)acryloyloxy(iso)propylene norbornanecarboxyamide, (meth)acryloyloxy(iso)butylene norbornanecarboxamide, (meth)acryloyloxy(iso)amylene norbornanecarboxamide and (meth)acryloyloxy(iso)hexylene norbomanecarboxamide; (meth)acryloyloxyalkylene norbornenecarboxamides such as (meth)acryloyloxymethylene norbornenecarboxamide,

(meth)acryloyloxyethylene norbornenecarboxamide, (meth)acryloyloxy(iso)propylene norbornenecarboxyamide, (meth)acryloyloxy(iso)butylene norbornenecarboxyamide, (meth)acryloyloxy(iso)amylene norbornenecarboxamide and (meth)acryloyloxy(iso)hexylene norbornenecarboxamide; (meth)acryloyloxyalkyleneoxa norbornanecarboxamides such as (meth)acryloyloxymethylene oxanorbomanecarboxamide, (meth)acryloyloxyethylene oxanorbornanecarboxamide, (meth)acryloyloxy(iso)propylene oxanorbornanecarboxacyamide, (meth)acryloyloxy(iso)butylene oxanorbomanecarboxyamide, (meth)acryloyloxy(iso)amylene oxanorbornanecarboxamide and (meth)acryloyloxy(iso)hexylene oxanorbomanecarboxamide; (meth)acryloyloxyalkylene oxanorb ornenecarb oxami de s such as (meth)acryloyloxymethylene oxanorbornenecarboxamide, (meth)acryloyloxyethylene oxanorbornenecarboxamide, (meth)acryloyloxy(iso)propylene oxanorbornenecarboxyamide, (meth)acryloyloxy(iso)butylene oxanorbornenecarboxyamide, (meth)acryloyloxy(iso)amylene oxanorbornenecarboxamide and (meth)acryloyloxy(iso)hexylene oxanorbornenecarboxamide; (meth)acryloyloxyalkylene (1 to 6 carbon atoms) norbornene-2-alkyl (1 to 6 carbon atoms)-2-carboxamide, (meth)acryloyloxyalkylene (1 to 6 carbon atoms) dicyclopentenylcarboxamide, (meth)acryloyloxyalkylene (1 to 6 carbon atoms) dicyclopentanylcarboxamide, (meth)acryloyloxyalkylene (carbon 1-6, alkyl (carbon 1-6), dicyclopentanyldicarboxamide, bis(meth)acryloyloxyalkylene (1 to 6 carbon atoms) dicyclopentanyldicarboxamide, (meth)acryloyloxyalkylene (1 to 6 carbon atoms) isobornanecarboxamide, bis(meth)acryloyloxyalkylene (1 to 6 carbon atoms) cyclohexanedicarboxamide, and the like.

The polymerizable compound (B) is a compound having one or more ethylenically unsaturated groups except for a (meth)acrylate (A). The ethylenically unsaturated group of B is one or more groups selected from (meth)acrylate group, (meth)acrylamide group, vinyl group, vinyl ether group, alkyl vinyl ether group, allyl group, (meth)allyl ether group and maleimide, and is preferably acrylate group and acrylamide group from the viewpoint of having high curability to active energy rays. The polymerizable compound (B) contains a polymerizable compound (b1) having one or more chain substituents having 1 to 36 carbon atoms and/or a polymerizable compound (b2) having one or more cyclic substituents having 3 to 20 carbon atoms (excluding A and b 1), and both b 1 and b2 are monofunctional polymerizable compounds having one ethylenically unsaturated group in the molecule (hereinafter also referred to as monofunctional monomers) or polyfunctional polymerizable compounds having two or more ethylenically unsaturated groups in the molecule (hereinafter also referred to as a polyfunctional monomer), and in addition to b 1 and b2, a polymerizable compound (b3) having one or more ethylenically unsaturated groups in the molecule may also be contained.

The active energy ray curable composition (E) according to the present embodiment contains a polymerizable compound (b1) or a polymerizable compound (b2), and preferably contains both b 1 and b2 from the viewpoint of further improving various properties. By containing b 1 and b2, E can be optimized according to various applications such as viscosity, pigment dispersibility and printability in inkjet ink compositions; shaping accuracy and strength of modeled objects in curable compositions for three-dimensional modeling; adhesion, reworkability and yellowing resistance in pressure-sensitive adhesive compositions; adhesive strength and impact resistance in adhesive compositions; wettability to substrates surface and hardness of the resulting coating film in coating compositions, and the like. From this viewpoint, the mass ratio of the contents of b 1 and b2 from 30/1 to 1/10 is more preferablely, and 10/1 to 1/5 is particularly preferred.

In the polymerizable compound (b 1) has one or more ethylenically unsaturated groups and chain substituents having 1 to 36 carbon atoms in the molecule, and the chain substituents may be linear or branched, saturated alkyl or unsaturated, and may have or be substituted with hydroxyl groups, primary amino groups, secondary amino groups, tertiary amino groups, thiol groups, ether groups, ester groups, ketone groups, carboxylic acid, amide groups, sulfonic acid, etc. The ethylenically unsaturated group of b 1 is one or more ethylenically unsaturated groups selected from the group consisting of (meth)acrylate group, (meth)acrylamide group, vinyl group, vinyl ether group, alkyl vinyl ether group, allyl group, (meth)allyl ether group and maleimide group, and from the viewpoint of having high curability against active energy rays, acrylamide groups and acrylate groups are preferable.

Among these b1 above, examples of the monofunctional polymerizable compounds having acrylamide groups include N-substituted alkyl acrylamide, N,N-disubstituted dialkyl acrylamide with straight or branched chains having 1 to 36 carbon atoms; N-substituted alkenyl acrylamide or N, N-disubstituted dialkenyl acrylamide with straight or branched chains having 1 to 36 carbon atoms; N, N-disubstituted alkylalkenyl acrylamide with straight or branched chains having 1 to 36 carbon atoms; the aforementioned various compounds having acrylamide groups which have or be substituted with one or more hydroxy groups, primary to tertiary amino groups, thiol groups, ether groups, ester groups, ketone groups, carboxylic acid groups, amide groups, sulfonic acid groups, etc. Among these, dimethylacrylamide, diethylacrylamide, isopropylacrylamide, t-butylacrylamide, ethylhexylacrylamide, n-octylacrylamide, t-octylacrylamide, oleyl acrylamide, methoxybutylacrylamide, butoxymethylacrylamide, N-(2-hydroxyethyl)acrylamide, N-(2-hydroxypropyl)acrylamide, N-(3-dimethylamino)propylacrylamide and diacetone acrylamide are preferable from the viewpoint of easy availability of industrial products. These monofunctional polymerizable compounds having an acrylamide may be used alone or in combination of two or more kinds thereof.

Among these b1 above, examples of the polyfunctional polymerizable compounds having acrylamide groups include monomer-type polyfunctional polymerizable compounds such as methylenebis(meth)acrylamide, ethylenebis(meth)acrylamide, diallyl(meth)acrylamide, N-tris(3-(meth)acrylamidepropoxymethyl)methyl-(meth)acrylamide, N, N-bis(2-(meth)acrylamide ethyl)(meth)acrylamide, 4,7,10-trioxa-1,13-tridecanbis(meth)acrylamide and N, N'-1,2-ethanedylbis(N-(2-(meth)acrylamide ethyl)(meth)acrylamide; oligomer-type polyfunctional polymerizable compounds such as polyurethane diacrylamide having no cyclic substituents. These polyfunctional polymerizable compounds having acrylamide groups may be used alone or in combination of two or more thereof.

Among these b 1 above, examples of the monofunctional polymerizable compounds having acrylate groups include alkyl acrylate with straight or branched chain having 1 to 36 carbon atoms, alkenyl acrylate with straight or branched chain having 1 to 36 carbon atoms, the aforementioned various compounds having acrylamide groups which have or be substituted with one or more hydroxy groups, primary to tertiary amino groups, thiol groups, ether groups, ester groups, ketone groups, carboxylic acid groups, amide groups, sulfonic acid groups, etc. Among these, butyl acrylate, 2-ethylhexylacrylate, stearyl acrylate, 2-(2-ethoxyethoxy)ethylacrylate, hydroxyethyl acrylate, 4-hydroxybutyl acrylate and the like are preferable from the viewpoint of easy availability of industrial products.

Among these b1 above, examples of the polyfunctional polymerizable compounds having acrylate groups include monomer-type polyfunctional polymerizable compounds such as ethylene glycol diacrylate, diethylene glycol diacrylate, methoxydipropylene glycol acrylate, methoxytripropylene glycol acrylate, 1,4-butanediol diacrylate, 1,6-hexanediol diacrylate, 1,6-hexanediol ethylene oxide modified diacrylate, hydroxypivalic acid neopentyl glycol diacrylate, glycerin diacrylate, pentaerythritol tetraacrylate, pentaerythritol triacrylate, pentaerythritol diacrylate, dipentaerythritol triacrylate, dipentaerythritol hexaacrylate, dipentaerythritol pentaacrylate, dipentaerythritol tetraacrylate, ethylene glycol diglycidyl ether diacrylate, diethylene glycol diglycidyl ether diacrylate, trimethylolethane triacrylate and trimethylolpropane triacrylate; oligomer-type polyfunctional polymerizable compounds such as polyether diacrylate such as polyethylene glycol diacrylate, polypropylene glycol diacrylate and polytetramethylene glycol diacrylate, polyurethane diacrylate, polyester diacrylate. These polyfunctional polymerizable compounds having acrylate groups may be used alone or in combination of two or more thereof.

In the polymerizable compound (b2) has one or more ethylenically unsaturated groups and one or more cyclic substituents having 3 to 20 carbon atoms in the molecule, and the cyclic substituents are monocyclic or multicyclic aliphatic rings, aromatic rings, aliphatic heterocycles, monocyclic or multicyclic aromatic heterocycles, and the aliphatic rings and aliphatic heterocyclic rings may be a saturated rings or an unsaturated rings. The ethylenically unsaturated group possessed by b2 is one or more groups selected from (meth)acrylate group, (meth)acrylamide group, vinyl group, vinyl ether group, alkyl vinyl ether group, allyl group, (meth)allyl ether group and maleimide group, and is preferably acrylate group and acrylamide group from the viewpoint of having high curability to active energy rays. In addition, when the ethylenically unsaturated group of b2 is a (meth)acrylate group, it does not have an amide group in the molecule. Further, when the ethylenically unsaturated group of b2 is a (meth)acrylamide group, it does not have a (meth)acrylate group in the molecule.

Among these b2 above, examples of the monofunctional polymerizable compounds having an acrylamide group include N-cyclohexyl acrylamide, N, N-dicyclohexyl acrylamide, N-cyclohexyl-N-methyl acrylamide, N-cyclohexyl-N-ethylacrylamide, N-cyclohexyl-N-propylacrylamide, N-cyclohexyl-N-butylacrylamide, N-phenylacrylamide, N-acryloylmorpholine, N-acryloylpiperidine, N-acryloyl-2-methylpiperidine, N-acryloyl-3-methylpiperidine, N-acryloyl-4-methylpiperidine, N-acryloyl-2,6-dimethylpiperidine, N-acryloyl-3,5 -dimethylpiperidine, N-acryloyl-3,3-dimethylpiperidine, N-acryloyl-4,4-dimethylpiperidine, N-acryloyl-2,2,6,6-tetramethylpiperidine, N-acryloyl-2-methyl-5-ethylpiperidine, N-acryloyl-4-methyl-4-ethylpiperidine, N-acryloyl-2-ethylpiperidine, N-acryloyl-3-ethylpiperidine, N-acryloyl-4-ethylpiperidine, N-acryloyl-2-propylpiperidine, N-acryloyl-3-propylpiperidine, N-acryloyl-4-propylpiperidine, N-acryloyl-3-isopropylpiperidine, N-acryloyl-4-isopropylpiperidine, N-acryloylhexamethyleneimine, N-acryloyl-2-methylhexamethyleneimine, N-acryloyl-3-methylhexamethyleneimine, N-acryloyl-4-methylhexamethyleneimine, N-acryloyl-2-ethylhexamethyleneimine, N-acryloyl-3-ethylhexamethyleneimine, N-acryloyl-4-ethylhexamethyleneimine, N-acryloyl-3-propylhexamethyleneimine, N-acryloyl-4-propylhexamethyleneimine, N-acryloyl-3-isopropylhexamethyleneimine, N-acryloyl-4-isopropylhexamethyleneimine, N-acryloyl-3,5-dimethylhexamethyleneimine, N-acryloyl-4,4-dimethylhexamethyleneimine, N-acryloylheptamethyleneimine, N-acryloyloctamethyleneimine, N-acryloyldecamethyleneimine, dopamine acrylamide, 3-acrylamidophenylboronic acid, and the like. Among these, N-cyclohexyl acrylamide, N, N-dicyclohexyl acrylamide, N-cyclohexyl-N-methyl acrylamide, N-acryloyl morpholine, N-acryloyl piperidine, N-acryloyl-2-methylpiperidine, N-acryloyl-4-methylpiperidine, N-acryloyl-2,6-dimethyl piperidine, N-acryloyl-3,5-dimethyl piperidine, N-phenyl acrylamide, dopamine acrylamide, and 3-acrylamide phenylboronic acid are preferable from the viewpoint of easy availability of industrial products.

Among these b2 above, examples of the polyfunctional polymerizable compounds having acrylamide groups include polyfunctional polymerizable compound having acrylamide groups such as an oligomer type polyurethane diacrylamide having cyclic substituents. These polyfunctional polymerizable compounds having acrylamide groups may be used alone or in combination of two or more kinds thereof.

Among these b2 above, examples of the monofunctional polymerizable compounds having acrylate groups include phenoxyethyl acrylate, phenoxydiethylene glycol acrylate, phenoxytetraethylene glycol acrylate, phenoxyhexaethylene glycol acrylate, cyclohexyl acrylate, tert-butylcyclohexyl acrylate, benzyl acrylate, dicyclopentanyl acrylate, dicyclopentenyl acrylate, bornyl acrylate, isobornyl acrylate, tetrahydrofurfuryl acrylate, 2-methyl-2-adamantyl (meth)acrylate, and the like. Among them, phenoxyethyl acrylate, cyclohexyl acrylate,tert-butyl cyclohexyl acrylate, dicyclopentanyl acrylate, dicyclopentenyl acrylate, isobornyl acrylate, tetrahydrofurfuryl acrylate are preferable from the viewpoint of easy availability of industrial products.

Among these b2 above, examples of the polyfunctional polymerizable compounds having acrylate groups include a monomer-type polyfunctional polymerizable compounds such as dicyclopentanyl diacrylate, caprolactone modified dicyclopentenyl diacrylate, tricyclodecanedimethanol diacrylate, ethylene oxide modified bisphenol type A diacrylate, propylene oxide modified bisphenol type A diacrylate and cyclohexanedimethanol diacrylate; an oligomer-type polyfunctional polymerizable compounds such as polyurethane diacrylate having cyclic substituents, polyester diacrylate having cyclic substituents and polyether diacrylate having cyclic substituents. These polyfunctional polymerizable compounds having acrylate groups may be used alone or in combination of two or more kinds thereof.

The amount of the monofunctional polymerizable compound in the active energy ray curable composition (E), including the (meth)acrylate (A) and the polymerizable compound (B), is preferably 5 to 99% by mass based on the total amount of E. The amount of the polyfunctional polymerizable compound in E, including A and B, is preferably 1 to 95% by mass based on the total amount of E. When the monofunctional polymerizable compound and the polyfunctional polymerizable compound in E are within these ranges, it is possible to easily combine the respective compounds having different structures according to the purpose, and E can be suitably used in various applications. From this viewpoint, the total amount of the monofunctional polymerizable compound in E is more preferably 10 to 90% by mass, and particularly preferably 20 to 80% by mass. The total amount of the polyfunctional polymerizable compounds in E is more preferably 5 to 90% by mass, particularly preferably 10 to 80% by mass.

A sixth embodiment of the present invention is an active energy ray curable composition (E) in which a volume shrinkage rate (curing shrinkage rate) generated in a curing process by active energy ray irradiation is 5% or less. Such E preferably contains 5% by mass or more of (meth)acrylate (A) and 60% by mass or less of a polyfunctional polymerizable compound as the polymerizable compound (B), and more preferably contains 8% by mass or more of A and 50% by mass or less of a polyfunctional polymerizable compound as B, based on the entire E. Furthermore, it is particularly preferable that 5% by mass or more of an oligomer type polyfunctional polymerizable compound and/or 2% by mass or more of a polymer type polyfunctional polymerizable compound are contained as B based on the entire E because impact resistance of the obtained cured product can be imparted while maintaining a low curing shrinkage rate.

A seventh embodiment of the present invention is an active energy ray curable composition (E) in which a saturated water absorption rate in a cured product of the active energy ray curable composition is 10% or less. Such E preferably contains 5% by mass or more of (meth)acrylate (A) and 20% by mass or more of a compound having chain or cyclic substituents having 6 or more carbon atoms as the polymerizable compound (B), and more preferably contains 10% by mass or more of A and 25% by mass or more of b1 or b2 having chain substituents having 6 or more carbon atoms as B. Furthermore, it is particularly preferable to contain 8% by mass or more of an oligomer-type compound having chain or cyclic substituents having 6 or more carbon atoms and/or 3% by mass or more of b1 or b2 having chain substituents having 6 or more carbon atoms as B based on the entire E because the water absorption of the obtained cured product tends to be low and the durability of the cured product tends to be improved at the same time.

An eighth embodiment of the present invention is an ink composition (hereinafter also referred to as an ink). The ink composition contains the active energy ray curable composition (E) according to the first to seventh embodiments. The ink composition preferably contains 3 to 50% by mass of (meth)acrylate (A) which is a constituent of E, 20 to 80% by mass of the polymerizable compound (b1), and 0 to 70% by mass of the polymerizable compound (b2). By containing A, the ink composition has high curability, and the surface drying property of the obtained printed surface is good. In addition, by containing b1, the viscosity of the ink composition can be easily adjusted, the dispersibility of pigments in the case of blending the pigments is good, and the ejection of the ink composition at the time of printing is highly stable. In a case where a polyfunctional compound is used as the b1, the ink composition has sufficient curability even when the b2 is not contained, while when the b2 is contained, both the curability of the ink composition and the surface-drying properties of the obtained printed surfaces are improved. Furthermore, by combining A, b1 and b2, the viscosity of the ink composition can be suitably adjusted according to the printing method, and the resulting printed surface is excellent in print definition. As a method for applying the ink composition to the substrate, a conventionally known method can be used. The viscosity of the ink composition at 25°C is preferably less than 500 mPa · s, and more preferably less than 100 mPa · s from the viewpoint that the ink composition can be applied to a substrate by an inkjet method. After the ink composition has been applied to the substrate, the ink is cured by irradiation with active energy rays to form a printing surface (ink layer or printing layer). From the viewpoint that the ink composition has good viscosity, pigment dispersibility, curability, and ejection stability, and the resulting printed surface has a good balance of printing properties such as surface dryness and sharpness, the ink composition preferably contains 8 to 45% by mass of A, 30 to 80% by mass of b1 and 5 to 65% by mass of b2, and more preferably contains 10 to 40% by mass of A, 40 to 60% by mass of b1 and 10 to 50% by mass of b2.

A ninth embodiment of the present invention is an ink composition for two dimensional or three-dimensional modeling (hereinafter also referred to as an ink composition for modeling or an ink for modeling). The ink composition

for modeling contains the active energy ray curable composition (E) according to the first to seventh embodiments. It is preferable that the ink composition for modeling contains 5 to 60% by mass of (meth)acrylate (A), 15 to 80% by mass of the polymerizable compound (b1), and 10 to 80% by mass of the polymerizable compound (b2), which are components of E. By containing compound A, the curing shrinkage of the ink composition for modeling is low, and therefore the shaping accuracy is good, and the strength and water resistance of the obtained modeled object are good, and in particular, the heat resistance and impact resistance of the modeled object are excellent. Since the viscosity of the ink composition for modeling can be easily adjusted by containing b 1, the ink composition for modeling is highly stable and highly accurate during modeling, and since the ink composition for modeling contains b2, the curability of the ink composition for modeling is high and the strength of the obtained modeled object is high. When a polyfunctional compound of oligomer type or polymer type is contained as the b2, both water resistance and impact resistance of the obtained modeled object are improved. Furthermore, by combining A, b1, and b2, it is possible to suitably adjust the viscosity of the ink composition for modeling according to the modeling method, and the shaping accuracy of the obtained modeled object is excellent. The ink composition for modeling is cured by irradiation with an active energy ray simultaneously with or immediately after being formed into a predetermined shape pattern to form a thin film, and the thin film is laminated to model a two dimensional or three-dimensional modeled object. The modeling method is not particularly limited, and examples thereof include an optical modeling method in which ejection is performed by an inkjet method and curing is performed by irradiation with an active energy ray. In this case, from the viewpoint of stable ejection, the viscosity of the ink composition for modeling at 25°C is preferably 1 to 200 mPa · s, and the ejection temperature is preferably in the range of 20 to 100°C. The ink composition for modeling preferably contains 5 to 50% by mass of A, 20 to 70% by mass of b1 and 15 to 75% by mass of b2. From the viewpoint that the obtained two dimensional or three-dimensional modeled object has heat resistance, impact resistance, water resistance, a good balance of high strength and excellent construction precision, the ink composition for modeling more preferably contains 10 to 40% by mass of A, 30 to 60% by mass of b1 and 20 to 60% by mass of b2.

A tenth embodiment of the present invention is a pressure-sensitive adhesive composition (hereinafter also referred to as a pressure-sensitive adhesive). The pressure-sensitive adhesive composition contains the active energy ray curable composition (E) according to the first to seventh embodiments. The (meth)acrylate (A) and the polymerizable compound (B) contained in the pressure-sensitive adhesive composition can be brought from E containing A and B, or can be directly added when the pressure-sensitive adhesive composition is prepared. In the pressure-sensitive adhesive composition, the total content of A is preferably 2 to 60% by mass, the total content of b 1 as B is preferably 5 to 75% by mass and the total content of b2 as B is preferably 10 to 80% by mass. By containing one or more amide groups and cyclic substituents, the pressure-sensitive adhesive composition has sufficient cohesive force and adhesive force, and it is excellent in adhesion to various substrates and stain resistance, and also it is excellent in durability of the resulting pressure-sensitive adhesive layer or pressure-sensitive adhesive laminate due to low curing shrinkage. In addition, by containing b 1 and/or b2, the viscosity and curability of the pressure-sensitive adhesive composition can be easily adjusted according to the purpose, and the pressure-sensitive adhesive composition and the pressure-sensitive adhesive layer and pressure-sensitive adhesive laminate obtained by curing the pressure-sensitive adhesive composition have good transparency. Particularly, when 5 to 50% by mass of an oligomer type or polymer type polyfunctional compound (total of b 1 and b2) is contained, the pressure-sensitive adhesive strength and durability of the pressure-sensitive adhesive layer after active energy ray curing are high, which is more preferably. On the other hand, by crosslinking the pressure-sensitive adhesive composition by the following crosslinking methods (1) to (3) using a crosslinking agent, it is also possible to obtain a pressure-sensitive adhesive layer and a pressure-sensitive adhesive laminate more excellent in stain resistance and durability.

Examples of the tenth embodiment crosslinking method include (1) a method in which the pressure-sensitive adhesive composition contains (meth)acrylate (A) and/or polymerizable compound (B) having a reactive functional group (hydroxyl group, amino group, carboxyl group, and oxazoline group) to introduce a crosslinking point, and further contains a compound (crosslinking agent) having a functional group (isocyanate group, carboxyl group and the like) which is reactable with the reactive functional group above, and then the pressure-sensitive adhesive composition is crosslinked by reaction with a crosslinking agent; (2) a method in which the pressure-sensitive adhesive composition contains a polyfunctional compound (monomer type, oligomer type or polymer type) and crosslink by active energy ray irradiation; and (3) examples include a method in which the pressure-sensitive adhesive composition contains a polyfunctional compound and A or B capable of introducing a crosslinking point, and is crosslinked by irradiation with active energy rays and a crosslinking reaction. Note that (3) is a method in which the crosslinking methods of (1) and (2) are appropriately combined. In the present invention, examples of the crosslinking agent include polyisocyanate having two or more isocyanate groups in the molecule, polyepoxy having two or more epoxy groups in the molecule, polyaziridine having two or more aziridine groups in the molecule, polycarboxylic acid having two or more carboxyl groups in the molecule, polyoxazoline having two or more oxazoline groups in the molecule, and the like. In the present invention, the crosslinking method in the other embodiments is the same.

The pressure-sensitive adhesive composition according to the tenth embodiment can form a pressure-sensitive

adhesive layer by being coated or molded on a separator or a substrate and then being cured by irradiation with active energy rays. When an organic solvent is contained in the pressure-sensitive adhesive composition, the pressure-sensitive adhesive composition may be coated or formed on a separator or a substrate, and cured while irradiating with active energy to evaporate (dry) the organic solvent, and it is preferable to perform active energy curing after drying by heating at a temperature of 60 to 120°C for 1 to 30 minutes because a pressure-sensitive adhesive layer having higher transparency can be obtained. As a method for applying the pressure-sensitive adhesive composition to the separator or the substrate, a conventionally known method can be used, and for example, an ordinary coating film forming method such as a spin coating method, a spray coating method, a knife coating method, a dipping method, a gravure roll method, a reverse roll method, a screenprinting method, or a bar coater method is used.

A laminate can be obtained by laminating a pressure-sensitive adhesive layer composed of the pressure-sensitive adhesive composition according to the tenth embodiment and various substrates. Examples of the method for laminating the pressure-sensitive adhesive layer and various substrates include a transfer method and a roll-to-roll method. The thickness of the pressure-sensitive adhesive layer in the laminate is not particularly limited because it varies depending on various applications, but is usually 4 to 150 μm, suitably about 20 to 120 μm in the case of use in automobile members, and about 30 to 100 μm in the case of use in electronic materials or optical members.

The substrates, depending on the purpose, may include various base materials having a wide range of polarity from low polarity to high polarity, such as organic base materials, inorganic base materials, and base materials made of organic-inorganic composite materials. Examples thereof include polyolefin resin such as polyethylene and polypropylene, polyester resin such as polyethyleneterephthalate and polycarbonate, ABS resin which is acrylonitrile-butadiene-styrene copolymer, polyimide resin, polyamide resin and acrylic resin such as poly(methyl methacrylate), metals such as steel, stainless steel, copper and aluminum, glass, a hybrid material in which inorganic material silica fine particles are dispersed in organic material polyimide. The applications of the obtained various laminates are not particularly limited, and examples thereof include electronic materials, optical members, and automobile members.

In the pressure-sensitive adhesive composition according to the tenth embodiment, the constituent (meth)acrylate (A) has a hydrophobic cyclic substituent exhibiting wettability to a low-polar substrates and a hydrophilic amide group exhibiting wettability to a high-polar substrates, and can impart good adhesion to a low-polar substrates to a high-polar substrates. In addition, high adhesive strength and stain resistance can be provided by developing strong cohesive force derived from hydrogen bonding between amide groups between molecules of A. Furthermore, since A has a high refraction index, A and the polymerizable compound (B) have good compatibility, and the pressure-sensitive adhesive composition has high transparency and yellowing resistance, the obtained adhesive layer also has high transparency and yellowing resistance, and is suitably used in the optical field such as an adhesive for an optical member and an adhesive sheet. Laminates comprising a pressure-sensitive adhesive layer having such properties and various substrates can be applied as pressure-sensitive adhesive films or pressure-sensitive adhesive sheets for electronic materials, optical members, and automobile members. When A has different ethylenically unsaturated groups, a part of the ethylenically unsaturated bonds can be subjected to a photocuring reaction by irradiation with active energy rays, and then the remaining ethylenically unsaturated bonds can be subjected to a thermal polymerization reaction by heating. In such a hybrid curing system, after an adhesive layer or an adhesive laminate is produced by an active energy ray, a crosslinking reaction further proceeds by heating, and peeling, reworking, recycling, and the like can be performed. In addition, by bonding with an opaque material such as metal and heating after irradiation with active energy rays, it is possible to achieve adhesiveness using active energy rays on translucent and opaque substrates, and adhesive laminates formed from dissimilar materials can also be obtained.

An eleventh embodiment of the present invention is an adhesive composition (hereinafter also referred to as an adhesive). The adhesive composition contains the active energy ray curable composition (E) according to any one of the first to seventh embodiments. In the present embodiment, a crosslinking agent can be contained, and crosslinking can be performed by various crosslinking methods described above. The adhesive composition, like the pressure-sensitive adhesive composition described above, can provide good adhesion from low-polar substrates to high-polar substrates by containing (meth)acrylate (A), can bond various substrates composed of an organic substrates, an inorganic substrates and an organic-inorganic composite material by various hardening methods by a combination of A and the polymerizable compound (B), can develop high adhesive strength and impact resistance in an adhesive layer after hardening and can be used as an adhesive composition for the same or different materials. Among various materials such as the resin material, the metal material, the glass material, and the hybrid material described above, two or more kinds of the same kind of materials refer to the same kind of materials, and two or more kinds of different kinds of materials refer to different kinds of materials.

The (meth)acrylate (A) and the polymerizable compound (B) contained in the adhesive composition can be brought from the active energy ray composition (E) containing A and B, or can be directly added when preparing the adhesive composition. The adhesive composition preferably has a total content of A of 2 to 65% by mass, a total content of b1 as B of 0 to 75% by mass, and a total content of b2 as B of 5 to 90% by mass. Because A contains one or more amide groups and cyclic substituents, the adhesive composition has sufficient cohesive force and adhesion to various

substrates, and the adhesive layer has high adhesive strength after curing, as well as the curing shrinkage of the adhesive composition is low, and the resulting adhesive layer and adhesive laminate have excellent impact resistance. Further, the viscosity of the adhesive composition and the content of the monofunctional polymerizable compound and the polyfunctional compound as B can be easily adjusted depending on the substrates and curing method to be used. When the content of the monofunctional polymerizable compound (total of b1 and b2) as B in the adhesive composition is 25 to 60% by mass, the viscosity of the adhesive composition can be easily adjusted, and cure shrinkage can be easily suppressed, which is preferable. Further, when the total content of the polyfunctional compound (total of b1 and b2) as B is from 15 to 60% by mass, the adhesive strength is high while maintaining high impact resistance, which is more preferably.

In the paint composition (hereinafter also referred to as a paint) according to the twelfth embodiment, the (meth)acrylate (A) and the polymerizable compound (B) contained in the paint composition can be taken in from the active energy ray composition (E) containing A and B, or can be directly added when preparing the paint composition. In the coating composition, the total content of A is preferably 5 to 60% by mass, the total content of b1 as B is preferably 5 to 65% by mass, and the total content of b2 as B is preferably 10 to 80% by mass. When A contains an amide group and a cyclic substituent, the wettability of the coating agent composition to various substrates is high, the abrasion resistance of the coating film after curing is high, the curing shrinkage of the coating composition is low, the surface of the obtained cured coating film does not generate unevenness, and the appearance is excellent. In addition, the viscosity of the coating composition and the contents of the monofunctional polymerizable compound and the polyfunctional compound as B can be easily adjusted according to the substrates to be used and the curing method. From the viewpoint of wide adjustment of the viscosity value of the coating composition from low viscosity to high viscosity, the content of the monofunctional polymerizable compound (total of b1 and b2) as B is preferably 0 to 60% by mass, and the content of the polymerizable compound of oligomer type or polymer type (total of b1 and b2) as B is preferably 5 to 40% by mass. Further, when the content of the polyfunctional compound (total of b1 and b2) as B is 15 to 85% by mass, it is more preferably because it has high wear resistance while maintaining the excellent appearance of the cured coating film. Further, in the two-stage irradiation of UV rays and EB rays as active energy rays, the monomers do not remain in the cured coating film, and the appearance of the cured coating film does not change (deteriorate) over time. Particularly, when the cyclic substituents of A have unsaturated bonds, the (meth)acrylate of A and the unsaturated bonds of the cyclic substituents can be photocured stepwise by UV rays and EB rays, and the abrasion resistance of the resulting cured coating film is further improved.

In the sealing composition according to the thirteenth embodiment, the (meth)acrylate (A) and the polymerizable compound (B) contained in the sealing composition can be brought from the active energy ray composition (E) containing A and B, or can be directly added when the sealing composition is prepared. In the sealing composition, the total content of A is preferably 5 to 60% by mass, the total content of b 1 as B is preferably 0 to 70% by mass, and the total content of b2 as B is preferably 10 to 70% by mass. When A contains an amide group and a cyclic substituent, the curing shrinkage of the sealing composition is low, and the obtained sealant has excellent heat-sealing resistance (heat cycle resistance). In addition, by containing b 1 and/or b2, it becomes easy to adjust the viscosity, the curing method, and the curing property of the sealing composition according to the purpose, and the sealant obtained by curing has a high sealing effect and good resistance to moist heat yellowing and corrosion resistance, which is preferable. Particularly, when the content of the oligomer type or polymer type polyfunctional compound (total of b1 and b2) is 2 to 45% by mass, the water absorption of the sealant after curing with active energy rays is low and the outgassing resistance is high, which is more preferably.

In the cosmetic for nails (nail cosmetic) according to the fourteenth embodiment, the (meth)acrylate (A) and the polymerizable compound (B) contained in the cosmetic for nails can be brought from the active energy ray composition (E) containing A and B, or can be directly added when the cosmetic for nails is prepared. In the nail cosmetic, the total content of A is preferably 1 to 30 % by mass, the total content of b1 as B is preferably 10 to 65 % by mass, and the total content of b2 as B is preferably 5 to 70 % by mass. When A contains an amide group and a cyclic substituent, curing shrinkage of the nail cosmetic is low, and adhesion and surface hardness of the obtained cured film are excellent. In addition, by containing b1 and/or b2, it becomes easy to adjust the viscosity, the curing method, and the curing property of the nail cosmetic according to the purpose, and the obtained nail cosmetic cured film has high surface gloss, which is more preferably.

In the decorative coating agent according to the fifteenth embodiment, the (meth)acrylate (A) and the polymerizable compound (B) contained in the decorative coating agent can be brought from the active energy ray composition (E) containing A and B, or can be directly added when the decorative coating agent is prepared. In the decorative coating agent, the total content of A is preferably 1 to 50% by mass, the total content of b1 as B is preferably 10 to 60% by mass, and the total content of b2 as B is preferably 15 to 70% by mass. When A contains an amide group and a cyclic substituent, the curing shrinkage of the decorative coating agent is low, and the obtained decorative coating film is excellent in elongation and bending resistance. In addition, by appropriately adjusting the content of b1 and/or b2, it is possible to increase the tack resistance and pencil hardness of the obtained decorative coating film. Furthermore, after curing with

active energy rays and/or after decorative molding such as stretching of the cured film, thermal polymerization of residual monomers by heat treatment, particularly in the case where the cyclic substituents of A has an unsaturated bond, the crosslinking rate of the decorative coating film by thermal polymerization of the unsaturated bond is increased, and the scratch resistance and sunscreen resistance of the coating film are further improved.

In the dental materials according to the sixteenth embodiment, the (meth)acrylate (A) and the polymerizable compound (B) contained in the dental materials can be brought from the active energy ray composition (E) containing A and B, or can be directly added when the dental materials are prepared. In the dental materials, the total content of A is preferably 0.5 to 50% by mass, the total content of b 1 as B is 5 to 70% by mass, and the total content of b2 as B is 10 to 60% by mass. When A contains an amide group and a cyclic substituent, the dental material has low curing shrinkage, and the obtained dental material cured product has excellent surface smoothness and adhesive strength. By appropriately adjusting the content of b 1 and/or b2, the solubility, dispersibility and curability of dental materials and the hardness of cured products can be enhanced. Furthermore, after curing with active energy rays, thermal polymerization of residual monomers by heat treatment, particularly in the case where the cyclic substituents of A has an unsaturated bond, the adhesive strength of the dental material cured product are further increased by thermal polymerization of the unsaturated bond.

The active energy ray curable compositions (E) according to the first to seventh embodiments of the present invention may contain various additives other than those described above as necessary. Examples of the additives include thermal polymerization inhibitors, antioxidants, ultraviolet sensitizers, antiseptics, phosphate and other flame retardants, surfactants, antistatic agents, colorants such as pigments and dyes, perfumes, antifoaming agents, fillers, silane coupling agents, surface tension-adjusting agents, plasticizers, surface lubricants, leveling agents, softeners, organic fillers, inorganic fillers, and silica particles. These additives may be used alone or in combination of two or more kinds thereof. The content of these additives is not particularly limited as long as it does not adversely affect the properties exhibited by various molded articles of E, but is preferably 5% by mass or less based on the total mass of E.

In the various compositions according to the eighth to sixteenth embodiments of the present invention, various additives other than those described above can be blended as necessary. Examples of the additives include thermal polymerization inhibitors, antioxidants, ultraviolet sensitizers, antiseptics, phosphate and other flame retardants, surfactants, antistatic agents, colorants such as pigments and dyes, perfumes, antifoaming agents, fillers, silane coupling agents, surface tension-adjusting agents, plasticizers, surface lubricants, leveling agents, softeners, organic fillers, inorganic fillers, and silica particles. These additives may be used alone or in combination of two or more kinds thereof. The content of these additives is not particularly limited as long as it does not adversely affect the properties exhibited by various molded articles obtained from various compositions, but is preferably 30% by mass or less based on the total mass of the composition. If necessary, water, an organic solvent or a mixture thereof can be used as a solvent or a diluent. The content of such a solvent is not particularly limited as long as it does not adversely affect the properties exhibited by various molded articles obtained from various compositions, but is preferably 95% by mass or less based on the total mass of the composition.

The active energy ray curable composition (E) may further contain a polymerization initiator. In this case, E has further improved curability by irradiation with active energy rays, and can be suitably used as an active energy ray curable ink composition, a two-dimensional or three-dimensional shaping composition, a pressure-sensitive adhesive composition, an adhesive composition, a coating composition, a sealant composition, a nail cosmetic, a decorative coating agent, a dental material, a water-resistant coating composition, a coating agent for vehicles, a coating agent for indoor and/or outdoor building materials, and the like according to another embodiment of the present invention.

In the present specification, the active energy ray is defined as an energy ray capable of decomposing an active species-generating compound (photopolymerization initiator) to generate an active species. Examples of such active energy rays include visible rays, ultraviolet rays, infrared rays, $\alpha$-ray, $\beta$-ray, $\gamma$-ray, X-rays, and electron beams (EB). When an electron beam is used as the active energy ray, a photopolymerization initiator may not be used. On the other hand, when ultraviolet light, visible light, or the like is used, a photopolymerization initiator is preferably used. The irradiation with the active energy ray is preferably performed in an inert gas atmosphere such as a nitrogen gas or a carbon dioxide gas, or in an atmosphere in which the oxygen concentration is lowered, but the curable compositions used for various applications containing the active energy ray curable compositions (E) and E according to each embodiment of the present invention have good curability because they have the (meth)acrylate (A) and the polymerizable compound (B), and can be sufficiently cured even in a normal air atmosphere. The irradiation temperature of the active energy ray is preferably 10°C to 200°C, and the irradiation time is preferably 1 second to 60 minutes.

The photopolymerization initiator may be a substance that generates radicals upon irradiation with active energy rays (i.e., a photoradical polymerization initiator). For example, benzoin, benzoin methyl ether, benzoin ethyl ether, benzoin isopropyl ether, benzoin isobutyl ether, benzoins such as anisole methyl ether, 4-(2-hydroxyethoxy)phenyl(2-hydroxy-2-propyl)ketone, $\alpha$-hydroxy-$\alpha$, $\alpha$'-dimethyl acetophenone, methoxy acetophenone, 2,2'-dimethoxy-2-phenyl acetophenone, 2-hydroxy-2-cyclohexyl acetophenone, 2,2-diethoxy acetophenone, 2,2-dimethoxy-2-phenyl acetophenone, 1-hydroxycyclohexylphenyl ketone, 4-phenoxy dichloroacetophenone, 4-tert-butyl-dichloroacetophenone such as

acetophenones, 2-hydroxy-2-methyl propiophenone, propiophenone such as 2-hydroxy-4'-isopropyl-2-methyl propiophenone, benzophenone, methylbenzophenone, p-chlorobenzophenone, benzophenone such as p-dimethylaminobenzofuranone, thioxanthone, 2-chlorothioxanthone, 2-Chill thioxanthone, 2-isopropyl thioxanthone, 2,4-dichlorothioxanthone, 2,4-diethyl thioxanthone, 2,4-diisopropyl thioxanthone, thioxanthone such as dodecyl thioxanthone, bis (2,4,6-trimethylbenzoyl)-phenylphosphine oxide, 2,4,6-trimethylbenzoyl diphenyl phosphine oxide, (bis(2,4,6-trimethylbenzoyl)-2,4-di-n-butoxy phenyl phosphine oxide, acylphosphine oxides such as bis (2,6-dimethoxy benzoyl)-2,4,4-trimethyl pentyl phenyl phosphine oxide, benzyl, dibenzosuberone, α-acyloxime esters and the like may be mentioned. Further, as a commercially available product, trade name Omnirad 1116, Omnirad 1173, Omnirad 184, Omnirad 369, Omnirad 500, Omnirad651, Omnirad 754, Omnirad 819, Omnirad 907, Omnirad 1300, Omnirad 1800, Omnirad 1870, Omnirad 2959, Omnirad 4265, Omnirad TPO and the like manufactured by IGM Resins B.V., and trade name Ubeclyl P36 and the like manufactured by UCB can be used. These photopolymerization initiators may be used alone or in combination of two or more thereof.

The content of the photopolymerization initiator is usually 0.1 to 30% by mass, preferably 0.5 to 20% by mass, and more preferably 0.5 to 3% by mass with respect to the total mass of the various curable compositions according to the embodiments of the present invention. When the content of the photopolymerization initiator is less than 0.1% by mass, sufficient curability may not be obtained, and when the content of the photopolymerization initiator exceeds 30% by mass, performance such as strength of the cured product may be deteriorated, depending on the type of the photopolymerization initiator, the type of the active energy ray, illuminance, and the like. In the present specification, a compound having functional groups having photopolymerization initiation function and ethylenically unsaturated groups in the molecule at the same time can be used as the polymerizable photopolymerization initiator. Depending on the number of ethylenically unsaturated groups contained therein, a monofunctional polymerizable photopolymerization initiator or a polyfunctional polymerizable photopolymerization initiator is obtained. When a polymerizable photopolymerization initiator is contained, it may be used alone or may be used in combination with a commercially available photopolymerization initiator.

The active energy ray curable composition (E) may further contain a non-polymerizable oligomer and/or a non-polymerizable polymer. In the present specification, those having no ethylenically unsaturated group in the molecule and having a weight average molecular weight (Mw) of 1,000 or more and less than 10,000 are classified as non-polymerizable oligomers, and those having an Mw of 10,000 or more are classified as non-polymerizable polymers. Examples of the non-polymerizable oligomer and non-polymerizable polymer include thermoplastic resins, rosin-based resins, and mixtures thereof. Examples of thermoplastics include (meth)acrylic resin, cyclic polyolefin resin, cellulose resin, polyester resin, polyurethane resin, polysulfonic acid resin, ABS resin, polycarbonate resin, polyamide resin, polyimide resin. Examples of the rosin-based resins include natural rosins such as gum rosins and modified rosin resin such as hydrogenated rosins obtained by modifying natural rosins, disproportionated rosins, rosin-modified phenolic resin, maleic acid modified rosin resin, maleated rosins, and esterified gums. These non-polymerizable oligomers and/or non-polymerizable polymers may be used alone or in combination of two or more kinds thereof.

The total content of the non-polymerizable oligomer and the non-polymerizable polymer is usually 0.1 to 20% by mass, preferably 0.5 to 15% by mass, and more preferably 1 to 10% by mass based on the total mass of the various curable compositions according to the embodiments of the present invention. By containing the non-polymerizable oligomer and the non-polymerizable polymer, the curing shrinkage rate of the active energy ray curable composition (E) and the curable composition used for each application containing the same may be reduced, the viscosity may be adjusted, the wettability to the substrates may be improved and the adhesion may be improved, and the effects of improving the surface hardness, tensile strength, water resistance, heat resistance, impact resistance durability, abrasion resistance and the like of the resulting cured film or cured product may be observed, and the composition may be suitably used according to the purpose.

## EXAMPLES

Hereinbelow, the present invention is described still more specifically by showing Examples and Comparative Examples; however, the present invention is not limited to these Examples. Abbreviations of constituent components described in Examples and Comparative Examples are as follows. In the following, all descriptions of "parts" and "%" are on a mass basis unless otherwise specified.

(1) (Meth)acrylates (A)

Table 1 shows the (meth)acrylate (A-1) to (A-15) used in the Examples.

(2) Polymerizable Compounds (B)

<Polymerizable compounds (b1) with chain substituents having 1 to 36 carbon atoms>

b1-1: Dimethyl acrylamide (registered trademarks "Kohshylmer" and "DMAA")
b1-2: Diethyl acrylamide (registered trademarks "Kohshylmer" and "DEAA")
b1-3: Isopropyl acrylamide (registered trademarks "Kohshylmer" and "NIPAM")
b1-4: N-(2-hydroxyethyl) acrylamide (registered trademarks "Kohshylmer" and "HEAA")
b1-5: N-(3-dimethylamino)propyl acrylamide (registered trademarks "Kohshylmer" and "DMAPAA")
b1-6: Diacetone acrylamide (registered trademark "Kohshylmer")
b1-7: 2-Ethylhexyl acrylate
b1-8: 2-(2-Ethoxyethoxy)ethyl acrylate
b1-9: 4-Hydroxybutyl acrylate
b1-10: Pentaerythritol triacrylate
b1-11: Dipentaerythritol hexaacrylate
b1-12: 1,6-Hexanediol diacrylate
b1-13: Tripropylene glycol diacrylate

<Polymerizable Compounds (b2) with Cyclic Substituents Having 3 to 20 Carbon Atoms>

b2-1: N-cyclohexyl acrylamide (registered trademark "Kohshylmer")
b2-2: N-acryloyl morpholine (registered trademarks "Kohshylmer" and "ACMO")
b2-3: N-phenyl acrylamide (registered trademark "Kohshylmer")
b2-4: Dopamine acrylamide (registered trademark "Kohshylmer")
b2-5: 3-Acrylamide phenylboronic acid (registered trademark "Kohshylmer")
b2-6: Tert-butyl cyclohexyl acrylate ("registered trademark "Kohshylmer")
b2-7: Isobornyl acrylate
b2-8: Tetrahydrofurfuryl acrylate
b2-9: Tricyclodecane dimethanol diacrylate
b2-10: UV-3000B (bifunctional urethane acrylate (UV, manufactured by Mitsubishi Chemical Corporation)
b2-11: UV-6640B (bifunctional urethane acrylate (UV, manufactured by Mitsubishi Chemical Corporation)
b2-12: Quick Cure7100 (UV curable urethane oligomer (registered trademark "Quick Cure", manufactured by KJ Chemicals Corporation)
b2-13: Quick Cure8100 (UV curable urethane oligomer (registered trademark "Quick Cure", manufactured by KJ Chemicals Corporation)

<Polymerizable Compounds (b3) (Excluding A, b1 and b2)>

b3-1: Glycidyl methacrylate
b3-2: 2-Vinyl-2-oxazoline
b3-3: Acrylonitrile
b3-4: Acrylic acid

(3) Photopolymerization Initiators (C)

C-1: Omnirad 184 (photopolymerization initiator, manufactured by IGM Resins B. V)
C-2: Omnirad 1173 (photopolymerization initiator, manufactured by IGM Resins B. V)
C-3: Omnirad TPO (photopolymerization initiator, manufactured by IGM Resins B. V)

(4) Other Components

D-1: Hydrogenated rosin (non-polymerizable polymer, Fire KE-359, manufactured by Arakawa Chemical Industries, Ltd)
D-2: Acrylic resin (non-polymerizable polymer, VS-1057, manufactured by Seiko PMC Corporation)
D-3: Blemmer (non-polymerizable oligomer, PME-4000, manufactured by NOF Corporation)
D-4: BYK-331 (leveling agent, polyether modified poly(dimethylsiloxane), manufactured by Byk Chemie)
D-5: Pigment dispersion liquid (NX-061 Green, manufactured by Dainichiseika Color & Chemicals Mfg. Co., Ltd.)
D-6: Hexamethylene diisocyanate
D-7: Inorganic filler (titanium oxide)

(5) Substrates

PP: Polypropylene plate and film
PC: Polycarbonate plate and film
PMMA: Polymethylmethacrylate plate and film
PET: Easy adhesion polyethylenetelephthalate plates and films
ABS: Acrylonitrile-butadiene-styrene copolymer synthetic resin plate
SPCC: Cold-rolled steel sheet
SUS: Stainless steel plate
Al: Aluminum plate
Cu: Copper plate
PO: Polyolefin sheet
PVC: Polyvinylchloride plate
HDF: Wood board (high-density fiberboard)

<Preparation and Evaluation of Active Energy ray curable Composition>

Examples 1 to 16 and Comparative Examples 1 to 4

The (meth)acrylate (A), the polymerizable compound (B) (b1 having chain substituents and b2 having cyclic substituents), the photopolymerization initiator (C), and other components used in the present embodiment were weighed in proportions shown in Table 2, and uniformly mixed at room temperature to prepare curable compositions of Examples and Comparative Examples. The transparency (compatibility), curability and curing shrinkage resistance of the obtained curable composition were evaluated by the following methods, and the results are shown in Table 2. The water resistance of the cured products obtained in Examples and Comparative Examples was evaluated by the following method, and the results are shown in Table 2.

<Evaluation of Transparency (Compatibility) of Curable Composition>

The state of the active energy ray curable composition prepared in each of Examples and Comparative Examples was visually observed, and transparency (compatibility) was evaluated in four grades.

◎: The transparency was high and no turbidity or separation was observed.
○: The transparency was high, but slight turbidity was observed.
△: There was no phase separation, but there was turbidity.
×: There was turbidity and phase separation.

<Evaluation of Curability (365 nm, 385 nm, and 405 nm)>

The active energy ray curable composition prepared in each of Examples and Comparative Examples was applied to an easy adhesion treated surface of a PET film ("Cosmoshine A-4100" manufactured by Toyobo Co., Ltd) having a thickness of 100 μm using a bar coater so as to have a film thickness of 20 μm, and then the coating film was cured by irradiation with ultraviolet rays. The cumulative amount of light at which the cured product was not tacked when touched was determined, and the curability was evaluated in four grades. The following three types of lamps 1) to 3) were used for ultraviolet irradiation. In addition, the lower the cumulative amount of light required until tack disappears (complete curing), the higher the curability.

1) UVLED lamp: wave 365 nm, illumination 100 $mW/cm^2$
2) UVLED lamp: wave 385 nm, illumination 100 $mW/cm^2$
3) UVLED lamp: wave 405 nm, illumination 100 $mW/cm^2$

◎: When the cumulative amount of light was less than 200 $mJ/cm^2$, the tack disappeared.
O: When the cumulative amount of light was 200 $mJ/cm^2$ or more and less than 500 $mJ/cm^2$, the tack disappeared.
△: When the cumulative amount of light was 500 $mJ/cm^2$ or more and less than 1000 $mJ/cm^2$, the tack disappeared.
×: When the cumulative amount of light was more than 1000 $mJ/cm^2$, the tack disappeared (including a case where tack did not disappear).

<Evaluation of Curing Shrinkage Resistance>

A silicone spacer (vertical 50 mm × horizontal 50 mm × thickness 5 mm) was set on a glass plate (vertical 30 mm × horizontal 15 mm × thickness 1 mm), and the active energy ray curable composition prepared in each of Examples and Comparative Examples was poured into the spacer and cured by ultraviolet irradiation (Apparatus: Inverter-type conveyor apparatus ECS-4011GX manufactured by Eye Graphics Co., Ltd., Metal Halide Lamp: M04-L41 manufactured by Eye Graphics Co., Ltd., Ultraviolet intensity: 700 $mW/cm^2$, cumulative amount of light: 1000 $mJ/cm^2$) to prepare a cured sheet.

The cure shrinkage rate was determined according to JIS K5600 2-4 by a change in densities of the active energy ray curable composition before and after curing as shown in the following calculation formula (1). The density of the curable composition before and after curing was measured according to JIS K7112 using an electron specific gravity meter (MDS-300 manufactured by Alfa Miller GmbH). The density of the cured sheet was thus produced was determined as the density after curing, and the following evaluation was performed from the curing shrinkage rate.

$$\text{Curing shrinkage rate (\%)} = (Ds - Dl) / Dl \times 100\,\% \quad \text{formula (1)}$$

Wherein Ds is the density of the curable composition after curing, and Dl is the density of the curable composition before curing)

◎: The curing shrinkage was 3% or less.
○: The curing shrinkage was more than 3% but 5% or less.
△: The curing shrinkage was more than 5% but 8% or less.
×: The curing shrinkage was more than 8%.

<Evaluation of Water Resistance of Cured Product>

A cured sheet was prepared in the same manner as in the evaluation of the curing shrinkage resistance described above, and the obtained sheet was cut into 3 cm squares, dried under vacuum at 60°C for 24 hours, and accurately weighed as a dried sheet to obtain the weight of the cured product in a dry state. The dried sheet was immersed in deionized water at 30°C, and after 24 hours and 48 hours, the weight immediately after removal from the deionized water was weighed to confirm that the sheet reached a saturated water absorption state, and the weight was defined as the weight of the cured product in a saturated water absorption state. The saturated water absorption was calculated according to the following formula, and the water resistance of the cured product was evaluated in four grades as follows.

Saturated water absorption rate (%) = (weight in saturated water absorption - weight in dry state) / weight in dry state × 100%

◎: The saturated water absorption was 8% or less.
O: The saturated water absorption was more than 8%, but was 10% or less.
△: The saturated water absorption was more than 10% but 20% or less.
×: The saturated water absorption was more than 20%.

As is clear from the results in Table 2, the active energy ray curable compositions of each Examples had good transparency (compatibility) and high curability to any of light rays of 365 nm, 385 nm, and 405 nm, and low curing shrinkage rate at the time of cuing because it had the (meth)acrylate (A) having one or more amide groups and cyclic substituents in the molecule and the polymerizable compound (b1) containing one or more chain substituents having 1 to 36 carbon atoms and/or the polymerizable compound (b2) containing one or more cyclic substituents having 3 to 20 carbon atoms. It was also confirmed that the obtained cured product had excellent water resistance. On the other hand, in Comparative Examples 1 to 4, since the (meth)acrylate (A) and the polymerizable compound (B) (b1 and/or b2) were not simultaneously contained, none of the curable compositions was satisfactory in all of transparency, curability and cure shrinkage resistance. In addition, the water resistance of the cured product of Comparative Example was poor. Such different properties between Examples and Comparative Examples are considered to be due to the interaction between A and B contained in the active energy ray curable composition of the present invention as described above.

<Preparation and Evaluation of Active Energy ray curable Ink Composition> Examples 17 to 22 and Comparative Examples 5 to 8

The active energy ray curable composition obtained in Table 2, the (meth)acrylate (A), the polymerizable compound (B), the photopolymerization initiator (C), and other components were weighed according to the proportions shown in Table 3, and uniformly mixed at room temperature to prepare ink compositions of Examples and Comparative Examples. Using the prepared ink composition, the viscosity was measured by the following method, and the pigment dispersibility in the case of containing a pigment dispersion was evaluated. The active energy ray curability of the ink composition and the surface drying property of the obtained cured film were evaluated, inkjet printing was further performed, and the ink ejection stability and the clearness of the printed matter were evaluated as printability. The results of these evaluations are summarized in Table 3. Example 18 is an example of curing by irradiation with electron beams (EB) instead of ultraviolet rays. As the EB irradiation apparatus, a Curetron EBC-200-AA3 manufactured by Nisshin High Voltage Co., Ltd. was used (acceleration voltage: 200 kV, irradiation dose: 20kGy).

<Viscosity Measurement and Evaluation>

The viscosity of the ink composition was measured in accordance with JIS K5600 2-3 using a cone-plate viscosimeter (RE550 viscosimeter manufactured by Toki Sangyo Co., Ltd.). The viscosity of the ink composition for inkjet printing was evaluated in four grades as follows.

◎: 5 or more and less than100 mPa· s
○: 100 or more and less than500 mPa· s
△: 500 or more and less than2000 mPa· s
×: 2000 mPa · s or more

<Evaluation of Pigment Dispersibility>

Using the prepared ink composition, the state of aggregation or precipitation of the pigment was visually observed immediately after preparation and after standing at room temperature for 2 months, and the pigment dispersibility was evaluated in four grades as follows.

◎: Neither aggregation nor precipitation of the pigment was observed immediately after preparation nor after standing for 2 months.
O: No precipitation was observed immediately after preparation, but slight precipitation of the pigment was observed after standing for 2 months.
△: Slight aggregation and/or precipitation of the pigment was observed immediately after preparation, and it was clearly observed after standing for 2 months.
×: Aggregation and/or precipitation of the pigment were clearly observed immediately after the preparation.

Method for preparing a printed matter by irradiation with active energy rays The obtained ink composition was applied to a PET film having a thickness of 100 μm by a bar coater (RDS12) (film thickness after drying: 10 μm), and cured by ultraviolet irradiation (inverter-type conveyor apparatus ECS-4011GX, metahalide lamp M04-L41, manufactured by Eye Graphics Co., Ltd.) to prepare a printed matter.

<Evaluation of Curability of Ink Composition>

When the printed matter was produced by the method described above, the cumulative amount of light until the ink composition was completely cured (non-sticky state) was measured to evaluate the curability.

◎: completely cured at less than 1000 $mJ/cm^2$
○: completely cured at 1000 or more and less than 2000 $mJ/cm^2$
△: completely cured at 2000 or more and less than 5000 $mJ/cm^2$
×: 5000 $mJ/cm^2$ or more was required until completely cured.

<Evaluation of Surface Drying Property>

The printed matter produced by the method described above was allowed to stand in an environment at a room temperature of 23°C and a relative atmospheric humidity of 50% for 5 minutes, high-quality paper was placed on the

printed surface, a load of 1 $kg/cm^2$ was applied for 1 minute, and the degree of transfer of the ink to the paper was evaluated.

◎: The ink dried and there was no transfer to the paper.
○: The ink dried and there was slight transfer to the paper.
△: The ink was almost dry and there was transfer to the paper.
×: The ink was hardly dried, and there was much transfer to paper.

Inkjet printing and evaluation of printability

The prepared ink composition was filled in a commercially available inkjet printer (Luxel Jet UV350GTW, manufactured by Fujifilm Co., Ltd.), a solid image was printed using coated paper, and the printability of the ink was evaluated by the following method.

<Evaluation of Ejection Stability>

The printed state of the printed matter obtained by inkjet printing was visually evaluated.

◎: Good printing was performed without nozzle omission.
○: Slight nozzle omission was observed.
△: There was nozzle omission in a wide range.
×: There was non-ej ection.

<Sharpness Evaluation>

The image clarity of the inkjet printed matter obtained from the ink composition containing the pigment was visually observed.

◎: Ink bleeding was not observed at all, and the image was clear.
○: There was almost no ink bleeding, and the image was good.
△: Ink bleeding was slightly observed.
×: Significant ink bleeding was observed.

The viscosity of the active energy ray curable ink composition of the present invention can be arbitrarily adjusted according to various printing methods such as inkjet printing, offset printing, screen printing, and flexographic printing. As is clear from the results in Table 3, the ink composition for inkjet printing can be prepared to have a low viscosity, and when a pigment is blended, the ink composition has high pigment dispersibility. The reason why such results can be obtained is that the compatibility between the (meth)acrylate (A) and the polymerizable compound (B) contained in Examples is extremely good, and the polymerizable compound (b1) having chain substituents and the polymerizable compound (b2) having cyclic substituents have many kinds from low viscosity to high viscosity and are easily combined with other components such as a pigment dispersant. In addition, by the combination of A with b1 and/or b2, the ink compositions of Examples had high curability, the obtained cured films had good surface drying properties, and the clarity of printed matter which is printability as an inkjet ink composition was good. On the other hand, the ink compositions of Comparative Examples did not satisfy the viscosity, pigment dispersibility, curability, surface drying properties, and inkjet printability.

<Preparation and Evaluation of Active Energy Ray curable Ink Composition for Three-dimensional Modeling>

Examples 23-29 and Comparative Example 9 and10

The active energy ray curable composition obtained in Table 2, the (meth)acrylate (A), the polymerizable compound (B), the photopolymerization initiator (C), and other components were weighed according to the proportions shown in Table 4, and uniformly mixed at room temperature to prepare three-dimensional modeling ink compositions of Examples and Comparative Examples. Using the ink compositions for three-dimensional shaping, three-dimensional modeled object was produced by the following method, the strength, heat resistance, and shaping accuracy of the obtained cured article were evaluated, and the evaluation results are shown in Table 4.

<Strength Assessment>

A heavy-release PET film (manufactured by Toyobo Co., Ltd., polyester film E7001) having a thickness of 75 μm was adhered to a horizontally placed glass plate, and a spacer having a thickness of 1 mm and an internal portion punched out in a No. 2 dumbbell shape in accordance with JIS K6251 was placed, the three-dimensional forming ink compositions obtained in Examples and Comparative Examples were filled inside the spacer, and a light-release PET film (manufactured by Toyobo Co., Ltd., polyester film E7002) having a thickness of 50 μm was further overlaid thereon, and ultraviolet rays (apparatus: inverter-type conveyor device ECS-4011GX manufactured by Eye Graphics Co., Ltd., metal halide lamp: M04-L41 manufactured by Eye Graphics Co., Ltd., ultraviolet illuminance of 200 $mW/cm^2$, and integrated light quantity of 1000 $mJ/cm^2$) were irradiated from both sides to cure the three-dimensional modeling ink composition. Thereafter, the release PET films on both sides were removed to obtain cured products for Examples and of cured test pieces for Comparative Examples. In accordance with JIS K7161, tensile strength was measured using a table-top precision universal tester (Autograph AGS-X, manufactured by Shimadzu Corporation) under conditions of a tensile speed of 10 mm/min and a chuck-to-chuck distance of 50 mm in a temperature environment of 25°C, and strength was evaluated according to the following criteria.

◎: Tensile strength was 40 MPa or more.
○: Tensile strength was 30 MPa or more and less than 40 MPa.
△: Tensile strength was 20 MPa or more and less than 30 MPa.
×: Tensile strength was less than 20 MPa.

<Heat Resistance Evaluation>

A cured product was prepared in the same manner as the test piece for the tensile test, and the glass transition temperature (Tg) of the cured product was measured using a differential scanning calorimeter (DSC-60plus, manufactured by Shimadzu Co., Ltd.). The heat resistance was evaluated based on the measured value of the glass transition temperature (Tg) of the cured product according to the following criteria.

○: Tg of the cured product was 80°C or higher.
△: Tg of the cured product was 40°C or more and less than 80°C.
×: Tg of the cured product was less than 40°C.

<Evaluation of Impact Resistance>

A heavy-release PET film (manufactured by Toyobo Co. Ltd., polyester film E7001) having a thickness of 75 μm was adhered to a horizontally placed glass plate, and a spacer having a thickness of 4 mm and an interior of 10×80 mm was installed, each of the three-dimensional modeling ink compositions obtained in Examples and Comparative Examples corresponding to a thickness of 4 mm was filled inside the spacer, and a light release PET film (manufactured by Toyobo Co., Ltd., polyester film E7002) having a thickness of 50 μm was further stacked thereon, and ultraviolet rays (apparatus: inverter-type conveyor device ECS-4011GX manufactured by Eye Graphics Co., Ltd., metal halide lamp: M04-L41 manufactured by Eye Graphics Co., Ltd., ultraviolet illuminance of 200 $mW/cm^2$, and integrated light quantity of 1000 $mJ/cm^2$) were irradiated from both sides to cure the ink composition. Thereafter, the release PET films on both sides were removed, and the cured products was further irradiated with ultraviolet rays at a predetermined integrated light amount (apparatus: manufactured by ITEC Corporation System, desktop batch-type UV-LED curing apparatus MUVBA-0.3 × 0.3 × 0.5, wave length 405 nm, illumination (UV-V) 50 $mW/cm^2$, integrated light amount 5,000 $mJ/cm^2$), post-curing was performed to cure completely. Thereafter, using the obtained cured product as a test piece, Izod impact strength (notched) was measured in accordance with JIS K-7110, and the impact resistance was evaluated as follows. An Izod-Charpy impact tester "Model No.195-R" manufactured by Manufacturing Institute of Corp. Yasuda Seiki. was used. The higher the impact strength, the higher the impact resistance.

◎: 40J/m or more
○: 30J/m or more and less than 40J/m
△: 20J/m or more and less than 30J/m
×: less than 20J/m

<Water Resistance Evaluation>

Using a spacer having a thickness of 10 mm and an inner of 10 cm × 1 cm, a cured product test piece for water

resistance evaluation having a length 10 cm, a width of 1 cm, and a thickness of 1 mm was prepared by the method of strength evaluation described above. After measuring the weight of the obtained test piece immediately after shaping, the test piece was immersed in a beaker containing 100 ml of water, and the weight after immersion was measured after one day. The water absorption was measured by substituting the weight before immersion and the weight after immersion into the following formula, and the water resistance was evaluated according to the following criteria. The lower the water absorption rate, the higher the water resistance.

◎: Water absorption was less than 2%.
○: Water absorption rate was 2% or more and less than 2.5%.
△: Water absorption rate was 2.5% or more and less than 3%.
×: Water absorption rate was 3% or more.

<Shaping Accuracy Evaluation>

A heavy-release PET film (manufactured by Toyobo Co., Ltd., polyester film E7001) having a thickness of 75 μm was adhered to a horizontally placed glass plate, and a spacer having a thickness of 10 mm and an interior of 10× 10 mm was installed, each of the three-dimensional modeling ink compositions obtained in Examples and Comparative Examples corresponding to a thickness of 1 mm was filled inside the spacer. Then, the surfaces were smoothed by holding at 60°C for 30 seconds, and the three-dimensional modeling ink compositions were cured by ultraviolet irradiation (apparatus: inverter-type conveyor ECS-4011GX manufactured by Eye Graphics Co., Ltd., metal halide lamp: M04-L41 manufactured by Eye Graphics Co., Ltd., ultraviolet illuminance 200 mW/$cm^2$). Thereafter, the three-dimensional shaping ink composition was filled to a 1 mm thickness and cured 10 times in total to obtain a cured product of 10 mm × 10 mm × 10 mm. The height of the obtained cured product was measured. Further, the side surface of the obtained cured product was visually observed. These results were combined, and modeling accuracy was evaluated according to the following criteria.

◎: The height was less than 10 mm ± 0.1 mm, and there was no unevenness on the side surface.
O : The height was 10 mm ± 0.1 mm or more and less than ± 0.2 mm, or these was slight unevenness on the side surface.
△: The height was 10 mm ± 0.2 mm or more and less than ± 0.3 mm, or these was some unevenness on the side surface.
×: The height was 10 mm is ± 0.3 mm or more, or these was obvious unevenness on the side surface.

As is clear from the results in Table 4, the ink compositions for three-dimensional modeling of Examples had excellent cure shrinkage resistance, three-dimensional photo modeled objects could be obtained with high shaping accuracy by using the ink compositions, heat resistance and impact resistance of the resulting modeled objects were very good, and sufficient strength and water resistance could be obtained by adjusting the kinds and contents of the (meth)acrylate (A) and the polymerizable compound (B). Modeled objects having such good characteristics were not obtained from the compositions of the Comparative Examples.

<Preparation and Evaluation of Adhesive Composition>

Examples 30 to 35 and Comparative Examples 11 and 12

The active energy ray curable composition obtained in Table 2, the (meth)acrylate (A), the polymerizable compound (B), the photopolymerization initiator (C), and other components were weighed according to the proportions shown in Table 5, and uniformly mixed at room temperature to prepare pressure-sensitive adhesive compositions of Examples and Comparative Examples. Using the pressure-sensitive adhesive composition, a pressure-sensitive adhesive layer and a pressure-sensitive adhesive sheet were prepared by the following method, and the curability of the pressure-sensitive adhesive composition, the adhesion to various substrates, and the transparency, adhesive strength, stain resistance (reworkability), durability and yellowing resistance of the obtained pressure-sensitive adhesive layer were evaluated, and the results are shown in Table 5. In Examples 30 and 33, the pressure-sensitive adhesive sheet after curing with the active energy ray was placed in a thermostatic machine at 80°C, and after completing the crosslinking reaction by aging for 48 hours, various characteristics of the pressure-sensitive adhesive layer were evaluated.

<Curability Evaluation of Pressure-Sensitive Adhesive>

A heavy-release PET film (manufactured by Toyobo Co., Ltd., polyester film E7001) having a thickness of 75

μm was adhered to a horizontally placed glass plate, and a spacer having a thickness of 1 mm and an interior of 60 mm × 100 mm was installed, each of the prepared active energy ray curable pressure-sensitive adhesive composition of Examples and Comparative Examples is filled inside the spacer, and a light release PET film (manufactured by Toyobo Co., Ltd., polyester film E7002) having a thickness of 50 μm was further stacked thereon, and irradiation is performed by a UVLED lamp having a wave length 385 nm and a illuminance of 200 mW/cm$^2$ so that an cumulative amount of light is 1000 mJ/cm$^2$ to cure the adhesive composition. Thereafter, release PET films on both sides were removed, and the obtained cured product (adhesive layer) was touched to evaluate the curability in three grades.

◦: A cured product that maintains its shape was obtained, and tackiness was observed when the cured product was touched, but there was no adhesion of the liquid uncured product.
△: A cured product that maintains its shape was obtained, and tackiness was observed when the cured product was touched, but there was adhesion of a liquid uncured product.
×: This is a state in which curing was insufficient, a cured product which maintains a shape could not be obtained, and adhesion of a liquid residue was observed in a large amount.

<Preparation of Pressure-sensitive Adhesive Sheet and Adhesion Evaluation>

The active energy ray curable pressure-sensitive adhesive composition prepared in the same manner as described above was applied onto various plate-shaped substrates (substrates), and the pressure-sensitive adhesive layer was laminated to a thickness of 50 μm using a table-top roll laminator (RSL-382S, manufactured by Royal Sovereign) to avoid air bubbles with a light-release separator (Silicone coated PET film), and then irradiated with ultraviolet rays (apparatus: inverter-type conveyor apparatus ECS 4011GX manufactured by Eye Graphics Co., Ltd., metal halide lamp: M04 L41 manufactured by Eye Graphics Co., Ltd., ultraviolet intensity: 700 mW/cm$^2$, cumulative light amount: 5000 mJ/cm$^2$). Thereafter, the light-release separator was peeled off to obtain a pressure-sensitive adhesive sheet composed of the pressure-sensitive adhesive layer and the substrate. Using the obtained pressure-sensitive adhesive sheet, 100 squares with 1 mm$^2$ were cross-cut with a cutter knife in accordance with JIS K 5600, a cellophane tape was attached, and the number of squares in which the pressure-sensitive adhesive layer remained on the substrate side when the tape was peeled off at once was counted, and the adhesion was evaluated according to the following criteria.

◎: 100 pieces without peeling
○: 95 to 99 pieces without peeling
△: 70 to 94 pieces without peeling
×: 0 to 69 pieces without peeling

< Adhesive Strength Evaluation>

Under the conditions of a temperature of 23°C and a relative humidity of 50%, the pressure-sensitive adhesive layer was transferred to various types of film-like or plate-like base materials, and pressure-bonded by reciprocating two times using a pressure-bonding roller having a weight of 2 kg, and left to stand under the same atmosphere for 30 minutes. Thereafter, 180° peel strength (N/25 mm) was measured at a peel rate of 300 mm/min in accordance with JIS Z0237 using a tensile tester (device name: Tensilon RTA-100 manufactured by ORIENTEC).

◎: 30 (N/25 mm) or more
○: 15 (N/25 mm) or more and less than 30 (N/25 mm)
△: 8 (N/25 mm) or more and less than 15 (N/25 mm)
×: less than 8 (N/25 mm)

<Transparency Evaluation of Adhesive Layer>

The total light transmittance of the glass substrate was measured in accordance with JIS K 7105 using a haze meter (NDH 2000, manufactured by Nippon Denshoku Industries Co., Ltd.). The pressure-sensitive adhesive layer was transferred to a glass substrate under conditions of a temperature of 23°C and a relative humidity of 50%, and the total light transmittance of the glass substrate and the pressure-sensitive adhesive layer was measured. Then, the transmittance of the pressure-sensitive adhesive layer itself was calculated by subtracting the transmittance of the glass plate, and the transparency was evaluated in four grades as follows.

◎: Transmission was 90% or more.
○: Transmission was 85% or more and less than 90%.

△: Transmission was 50% or more and less than 85%.
×: Transmission was less than 50%.

<Stain Resistance (Reworkability) Evaluation>

A pressure-sensitive adhesive sheet was prepared in the same manner as in the measurement of the pressure-sensitive adhesive strength described above, allowed to stand at 80°C for 24 hours, and then the pressure-sensitive adhesive layer was peeled off, and contamination (remaining state of the pressure-sensitive adhesive layer (glue)) on the surface of the substrate film was visually observed.

◎: Transmission was 90% or more.
○: Transmission was 85% or more and less than 90%.
△: Transmission was 50% or more and less than 85%.
×: Transmission was less than 50%.

<Yellowing Resistance Evaluation >

A pressure-sensitive adhesive sheet was prepared in the same manner as in the measurement of the pressure-sensitive adhesive strength, set in a xenon fade meter (SC-700-WA: manufactured by Suga Test Instruments), irradiated with ultraviolet rays having an intensity of 70 $mW/cm^2$ for 120 hours, and then visually observed for discoloration of the pressure-sensitive adhesive layer on the pressure-sensitive adhesive sheet.

◎: Yellowing could not be visually observed at all.
○: Very slight yellowing could be visually observed.
△: Slight yellowing could be visually observed.
×: A clear yellowing could be visually observed.

<Durability Evaluation>

A pressure-sensitive adhesive sheet was prepared in the same manner as in the measurement of the pressure-sensitive adhesive strength, and after standing for 100 hours under conditions of a temperature of 85°C and a relative humidity of 85%, the presence or absence of lifting or peeling of the pressure-sensitive adhesive layer, bubbles and cloudiness was visually observed and evaluated according to the following criteria.

◎: It was transparent, and neither lifting nor peeling nor bubbles was observed.
◦: There was negligible fogging but no floating or peeling or air bubbles.
△: There was slight fogging or floating or peeling and air bubbles.
×: It was clouding, lifting, or flaking, and bubbles was observed.

As is clear from the results in Table 5, the active energy ray curable pressure-sensitive adhesive composition of Example had high curability, and the pressure-sensitive adhesive layer obtained by curing the composition had high transparency and good adhesiveness to various materials and adhesiveness (adhesive strength). In particular, since the cured product (adhesive layer) obtained from the active energy ray curable pressure-sensitive adhesive composition of the present invention contains (meth)acrylate (A) having one or more amide groups and cyclic substituents in combination as a constituent component, the stain resistance and yellowing resistance and durability of the cured product when the cured product was peeled off from the substrate were excellent. On the other hand, it was found that the composition of Comparative Example had low curability, transparency, adhesiveness to various materials, and adhesiveness, and the cured product had low stain resistance, yellowing resistance, and durability. In addition, due to the combined use of the crosslinking reaction, the adhesive strength between the obtained pressure-sensitive adhesive layer and various substrates was high. In the composition of Comparative Example and the cured product, such good properties were not obtained.

<Preparation and Evaluation of Adhesive Composition>

Examples 36 to 41 and Comparative Examples 13 and 14

The active energy ray curable composition obtained in Table 2, the (meth)acrylate (A), the polymerizable compound (B), the photopolymerization initiator (C), and other components were weighed according to the proportions

shown in Table 6, and uniformly mixed at room temperature to prepare adhesive compositions of Examples and Comparative Examples. Using the adhesive composition, the same type or different type of plate-like base material was adhered by the following method to prepare an adhesive test piece, and the adhesive strength and impact resistance were evaluated, and the results are shown in Table 6.

The adhesive composition was uniformly applied to one arbitrary sheet using two sheets of plate-like substrates of the same type or different types having a length of 100 mm, a width of 25 mm, and a thickness of 1 mm. When a solvent was contained in the adhesive composition, the mixture was applied in a large amount so that the thickness after drying was about the same as that in the case of no solvent, and dried at 90°C for 2 minutes. Thereafter, in accordance with JIS K 6850, the other one of the plate-like base materials was placed on the adhesive composition after coating, and the adhesive composition was bonded so that the overlapping region was 12.5 mm in length and 25 mm in width, and the thickness of the adhesive layer was adjusted to 100 μm by using a spacer, thereby preparing a bonded test piece. Then, UV (Ultraviolet irradiation device: inverter-type conveyor device ECS-4011GX manufactured by Eye Graphics Co., Ltd., metal halide lamp: M04-L41 manufactured by Eye Graphics Co., Ltd., ultraviolet illuminance: 700 $mW/cm^2$, integrated light quantity: $5000mJ/cm^2$ ) or EB (EB irradiation device: Nisshin High Voltage Cuatron EBC-200-AA3, accelerating voltage: 200 kV, irradiation dose: 20kGy) irradiation was performed from the upper surface of the bonded transparent or translucent substrate. In Examples in which UV and EB were described as curing methods in Table 6, test pieces irradiated with UV rays and EB rays were used as adhesive test pieces. In Examples in which UV heat was described as a curing method, a test piece irradiated with UV rays was further heated at 80°C for 48 hours, and the obtained test piece was used as an adhesive test piece.

<Adhesive Strength Evaluation>

Using the obtained adhesive test piece, tensile shear strength was measured under the condition of a tensile speed of 10 mm/min using Tensilon RTA-100 (manufactured by ORIENTEC) as a tester in accordance with JIS K 6850. The higher the tensile shear strength, the higher the adhesive strength.

◎: Tensile shear strength was 20 MPa or more.
○: Tensile shear strength was 15 MPa or more and less than 20 MPa.
△: Tensile shear strength was 10 MPa or more and less than 15 MPa.
×: Tensile shear strength was less than 10 MPa.

<Impact Resistance Evaluation >

Using the adhesive test piece obtained in the same manner as described above, the impact peel adhesion strength was measured using an impact tester No. 511 (manufactured by Maize Test Instruments Co., Ltd.) in accordance with JIS K6855. The higher the peel adhesion strength of the impact, the higher the impact resistance.

◎: Impact peel strength was 20 $KJ/m^2$ or more.
○: Impact peel strength was 15 $KJ/m^2$ or more and less than 20 $KJ/m^2$.
△: Impact peel strength was 10 $KJ/m^2$ or more and less than 15 $KJ/m^2$.
×: Impact peel strength was less than 10 $KJ/m^2$.

As is clear from the results in Table 6, the active energy ray curable adhesive composition of Example was easily cured by ultraviolet rays (UV) and electron beams (EB), and a laminate bonded by an adhesive layer having high adhesive strength and impact resistance could be obtained. By further heating after UV or EB irradiation, thermal curing continuously progresses, and is also applied to adhesion of an opaque substrate or a heterogeneous material. In particular, when the cyclic substituents of the (meth)acrylate (A) has an unsaturated bond, photopolymerization by an active energy ray proceeds at a high speed, so that the unsaturated bond of the cyclic substituents is likely to remain, and the unsaturated bond of the cyclic substituents can be completely polymerized by the subsequent thermal polymerization, and the adhesion and the impact resistance are further improved. In the composition of Comparative Example and the cured product, such good properties were not obtained.

<Preparation and Evaluation of Coating Composition>

Examples 42 to 47 and Comparative Examples 15 and 16

The active energy ray curable composition obtained in Table 2, the (meth)acrylate (A), the polymerizable compound (B), the photopolymerization initiator (C), and other components were weighed in proportions shown in Table

7, and uniformly mixed at room temperature to prepare coating compositions of Examples and Comparative Examples. The coating composition was applied onto various substrates, and the wettability of the coating composition to the various substrates was evaluated. In addition, the coating composition was applied to an ABS plate, UV, EB, or UV was further subjected to EB curing, the curability of the coating composition was evaluated, the friction resistance of the obtained cured coating film was evaluated, and the results are shown in Table 7.

<Wettability Evaluation>

The obtained various coating compositions were applied to various substrates with a bar coater (RDS 3), and cissing of the coating films was visually observed, and wettability was evaluated in four grades as follows.

◎: There was no cissing and the coating film was uniform.
◦: There was cissing is very slight, but the coating was nearly uniform.
△: There was some cissing, and the coating was substantially uniform as a whole.
×: There is a lot of cissing, and the coating was non-uniform.

<Appearance Evaluation of Cured Coating Film>

Each of the obtained coating compositions was coated on an ABS resin plate having a thickness of 3 mm by a bar coater (RDS 6) so that the film thickness after curing became 25 μm. UV irradiation (UV irradiation apparatus: inverter-type conveyor apparatus ECS-4011GX manufactured by Eye Graphics Co., Ltd., metal halide lamp: M04-L41 manufactured by Eye Graphics Co., Ltd., UV intensity: 700 $mW/cm^2$, cumulative light amount: 2000 $mJ/cm^2$) or EB (EB irradiation apparatus: Curetron EBC-200-AA3 manufactured by Nisshin High Voltage Co., Ltd., acceleration voltage: 200 kV, irradiation dose: 20kGy) was performed. In Table 7, the examples described as "EB after UV" as the curing method were irradiated with UV rays and then further irradiated with EB rays. The obtained cured coating film was touched with a finger, and the state of adhesion of the coating composition to the finger was confirmed to evaluate curability. Further, the surface smoothness of the cured coating film and the transparency of the coating film were evaluated by visual observation. The evaluation results are shown in Table 7. In the appearance evaluation, when a pigment was contained, only the surface smoothness of the cured coating film was evaluated.

Curability:

◦: Coating composition did not adhere.
△: Coating composition slightly adhered.
×: Coating composition adhered.

Coating film appearance:

◎: The surface was smooth and the coating film was transparent.
◦: The surface was smooth, and the coating film was entirely transparent with a slight white turbid portion.
△: The surface was uneven or the coating film had a white turbid portion.
×: The surface was uneven, and the coating film was cloudy.

<Evaluation of Abrasion Resistance>

The obtained cured coating film was evaluated for abrasion resistance in accordance with ISO20566 using a vehicle wash abrasion resistance tester (manufactured by Amtec Kistler GmbH, Amtec laboratory vehicle wash apparatus). The 20° gloss of the cured coating film before and after the abrasion test was measured with a gloss meter manufactured by BYK Gardner Co., and the gloss retention rate was calculated according to the following formula, and the abrasion resistance of the coating film was evaluated according to the following criteria. The higher the gloss retention rate, the higher the abrasion resistance of the cured coating film.

$$\text{Gloss retention (\%)} = \text{(gloss value after rubbing)} / \text{(gloss value before rubbing)} \times 100\%$$

Regarding the obtained gloss retention rate,

O: Gloss retention rate was 80% or more.
△: Gloss retention rate was 60% or more and less than 80%.
×: Gloss retention rate was less than 60%.

As is clear from the results of Table 7, the active energy ray curable coating compositions of Examples had good wettability to various substrates, had excellent curability, and the obtained cured coating films had good appearance and abrasion resistance. On the other hand, the coating compositions of Comparative Examples had low curability, and the appearance and abrasion resistance of the obtained cured films were also low. The coating compositions of the present invention have good wettability to general-purpose plastics, woods, and metals, have high curability to active energy rays, give cured coating films having good properties, and can be suitably used in a wide range as coatings for various electronic parts, indoor and outdoor coatings, floor coatings, vehicle coatings, and the like.

<Preparation and Evaluation of Active Energy Ray curable Sealing Compositions>

Examples 48 to 52 and Comparative Examples 17 and 18

The active energy ray curable composition obtained in Table 2, the (meth)acrylate (A), the polymerizable compound (B), the photopolymerization initiator (C), and other components were weighed in proportions shown in Table 8 and uniformly mixed at room temperature to prepare sealing compositions of Examples and Comparative Examples. Using the obtained sealing composition, a cured sealant (sealant cured product) was prepared by the following method, and properties were evaluated.

<Preparation of Sealant Cured Product>

A silicone spacer (vertical 50 mm × horizontal 50 mm × thickness 5 mm) was set on a glass plate (vertical 30 mm × horizontal 15 mm × thickness 3 mm), a copper foil (vertical 5 mm × horizontal 50 m × thickness 80 μm) was placed inside the spacer, and the prepared active energy ray curable sealing composition was injected. After sufficient deaeration, UV irradiation (apparatus: inverter-type conveyor ECS-4011GX manufactured by Eye Graphics Co., Ltd., metal halide lamp: M04-L41 manufactured by Eye Graphics Co., Ltd., UV illumination: 700 mW/cm$^2$, cumulative light amount: 1000 mJ/cm$^2$ to obtain a sealant cured product. The properties of the obtained cured product were evaluated by the following methods, and the results are shown in Table 8.

<Transparency Evaluation of Sealant Cured Product>

The obtained sealant cured product was allowed to stand in an atmosphere at a temperature of 23°C and a relative humidity of 50% for 24 hours, and then the transmittance of the cured product was measured with a haze meter (NDH-2000, manufactured by Nippon Denshoku Industries Co., Ltd.), and the transparency was evaluated in four grades as follows.

◎: Transmission was 90% or more.
○: Transmission was 85% or more and less than 90%.
△: Transmission was 50% or more and less than 85%.
×: Transmission was less than 50%.

<Evaluation of Yellowing Resistance to Heat and Humidity>

The obtained sealant cured product was allowed to stand for 24 hours in an atmosphere at a temperature of 23°C and a relative humidity of 50%, and then the transmission spectrum of the cured product was measured with a dedicated transmission color measuring machine (TZ-6000, manufactured by Nippon Denshoku Industries Co., Ltd.), and the initial b value was determined. Thereafter, the cured product was allowed to stand in a thermo-hygrostat set at 85°C and a relative humidity of 85% for 500 hours, and an accelerated test of resistance to yellowing due to heat and humidity was performed. The cured product after the test was similarly allowed to stand for 24 hours in an atmosphere at a temperature of 23°C and a relative humidity of 50%, and the transmitted color was measured to obtain the b value after wet heat. The difference between the b value after moist heat and the initial b value was defined as a change value Δ b (Δ b = b value after moist heat - initial b value). The yellowing resistance to heat and humidity of the cured product was evaluated in four grades as follows.

◎: The initial b value and the b value after wet heat were both 0.2 or less, and Δ b was 0.1 or less.
O: Any one or both of the initial b value and the b value after moist heat were more than 0.2, but both were 0.5 or less, and Δ b was 0.2 or less.
△: Any one or both of the initial b value and the b value after moist heat were more than 0.5, but both were 1.0 or less, and Δ b was 0.3 or less.
×: One or both of the initial b value and the b value after moist heat were more than 1.0, or Δ b was more than 0.3.

<Water Resistance Evaluation of Sealant Cured Product>

1 g of the obtained cured product was cut out and set as a test piece in a thermo-hygrostat at a temperature of 85°C and a relative humidity of 95%. After standing the test piece for 48 hours, the weight was measured again, and the water absorption rate was calculated by the following formula. The water resistance was evaluated in four grades as follows. The lower the water absorption, the higher the water resistance of the cured product.

Water absorption rate (%) = (weight after water absorption - weight before water absorption)/weight before water absorption × 100%

◎: Water absorption was less than 1.0%.
○: Water absorption was 1.0% or more and less than 2.0%.
△: Water absorption rate was 2.0% or more and less than 3.0%.
×: Water absorption rate was 3.0% or more.

<Outgassing Resistance Evaluation>

1 g of the obtained cured product was cut out and left to stand as a test piece in a thermostatic chamber set at a temperature of 100°C, a dry nitrogen stream was allowed to flow for 24 hours, the weight of the test piece was measured again, the generation rate of outgassing was calculated by the following formula, and evaluation was performed in four grades. The lower the generation rate of outgas, the higher the outgas resistance.

Outgassing generation rate (%) = (weight after test - weight before test)/weight before test × 100%

◎: Outgassing generation rate was less than 0.1%.
○: Outgassing generation rate was 0.1% or more and less than 0.3%.
△: Outgassing generation rate was 0.3% or more and less than 1.0%.
×: Outgassing generation rate was 1.0% or more.

<Evaluation of Heat Cycle Resistance>

The resulting cured product was allowed to stand at -40°C for 30 minutes and then at 100°C for 30 minutes as one cycle, and this cycle was repeated 100 times. The state of the cured product was visually observed, and the heat cycle resistance was evaluated in four grades.

◎: No change in was observed.
○: Slight bubble generation was observed, but crack generation was not observed. The cured product was transparent.
△: Some bubbles or cracks were observed and the cured product was slightly cloudy.
×: Bubbles or cracks occur over the entire surface and the cured product was in a translucent state.

<Corrosion Resistance Evaluation>

After the moisture and heat yellowing test, the surface of the copper foil was visually observed, and the corrosion resistance of the cured product was evaluated in four grades.

◎: no corrosion

◦: slight corrosion
△: some corrosion
×: significant corrosion

As is clear from the results in Table 8, the sealant cured products obtained from the active energy ray curable sealant compositions of Examples were high in transparency and water resistance, generated less outgassing, and excellent in moist heat yellowing resistance and metal corrosion resistance (corrosion resistance). On the other hand, these properties were not observed in the cured products obtained from the curable compositions of Comparative Examples. The sealing composition of the present invention can be suitably used as a sealant for optical members, electric devices, and the like.

<Preparation and Evaluation of Active Energy ray curable Nail Cosmetic>

Examples 53 to 58 and Comparative Examples 19 and 20

The active energy ray curable composition obtained in Table 2, the (meth)acrylate (A), the polymerizable compound (B), the photopolymerization initiator (C), and other components were weighed in proportions shown in Table 9, and uniformly mixed at room temperature to prepare nail cosmetics of Examples and Comparative Examples. Using the obtained nail cosmetic, curability and properties of the obtained cured film were evaluated by the following methods.

<Curability Evaluation of Nail Cosmetic>

Each of the active energy ray curable nail cosmetics prepared in Examples and Comparative Examples was applied onto a nylon 6 test piece ("SHT-NI6 (NC)" manufactured by Toray Plastics Precision Co., Ltd.) using a bar coater so as to have a film thickness of 10 μm, and then irradiated with ultraviolet rays using a gel nail-dedicated UVLED lamp (manufactured by Beauty Nylor Co., Ltd., wavelength: 405 nm, 48 W), and the time during which the tack disappears when touching the surface of the cured film was evaluated in four grades. The shorter the time required to eliminate the tack, the higher the curability.

◎: Tack disappeared in less than 1 minute.
○: Tack disappeared in 1 minute or more and less than 3 minutes.
△: Tack disappeared in 3 minutes or more and less than 10 minutes.
×: The tack did not disappear even after 10 minutes or more.

<Adhesion Evaluation (Nylon Substrate)>

Each of the obtained photocurable nail cosmetics of Examples and Comparative Examples was applied onto a nylon 6 test piece in the same manner as described above and irradiated with light for 3 minutes to prepare a cured film. On the surface of the obtained cured films 100 squares with 1 mm2 were crosscut with a cutter knife in accordance with JIS K 5600, and the number of squares remaining on the test piece when a commercially available cellophane tape was bonded and then peeled off was evaluated in four grades. The greater the number of squares remaining on the test piece, the higher the adhesion.

◎: The number of remaining squares was 100.
○: The number of remaining squares was 90 to 99.
△: The number of remaining squares was 60 to 89.
×: The number of remaining squares was less than 60.

<Surface Hardness Evaluation of Cured Film>

Cured films of Examples and Comparative Examples were prepared in the same manner as in the evaluation of adhesion, and the surface of the obtained film was pulled with a pencil having a hardness of HB by pressing a load of 750 g, and the occurrence of peeling and the presence of scratches were visually confirmed and evaluated in three grades. The smaller the occurrence of scratches or peeling, the higher the surface hardness.

O: No scratches or peeling occurred. Surface hardness was greater than pencil hardness HB.
△: Peeling did not occur, but scratches occurred.
×: Peeling occurred.

<Surface Glossiness Evaluation>

Cured films of Examples and Comparative Examples were prepared in the same manner as in the evaluation of adhesion, and the gloss of the surface of the film was visually observed and evaluated in three grades.

O: The gloss was confirmed.
△: Although reflection of light could be confirmed, cloudiness was observed.
×: Reflection of light could not be confirmed, and there was no gloss.

As is clear from the results of Table 9, the active energy ray curable nail cosmetics of Examples had excellent curability with respect to a commercially available UV lamp dedicated to gel nails, and also had high adhesion to a nylon base material (a material having a large number of amide bonds similar to nails which are a protein main component). From these results, it is understood that the active energy ray curable nail cosmetic of the present invention can be suitably used as a gel nail for a base gel to be directly applied to a nail. Further, the obtained cured film has good surface hardness and surface glossiness, and can be suitably used as a gel nail for top coating. On the other hand, the curable compositions of Comparative Examples had low curability, and the surface hardness and surface glossiness of the cured films were low.

<Preparation and Evaluation of Active Energy ray curable Decorative Coating Agent>

Examples 59-63 and Comparative Example 21 and 22

The active energy ray curable composition obtained in Table 2, the (meth)acrylate (A), the polymerizable compound (B), the photopolymerization initiator (C), and other components were weighed in proportions shown in Table 10 and uniformly mixed at room temperature to prepare decorative coating agents (active energy ray curable compositions used for decorative molding and decorative processing of decorative films, decorative sheets, and the like) of Examples and Comparative Examples. Using the obtained decorative coating agent, a laminate was produced by the following decorative processing, and properties of the obtained laminate were evaluated.

<Production of Laminate>

The obtained decorative coating agent was applied onto a 180 μm-thick polycarbonate film ("Panlite PC-2151 " manufactured by Teijin Co., Ltd.) using a bar coater (RDS 6) so as to have a thickness of 5 μm after drying, heated at 80°C for 3 minutes, and cured by ultraviolet irradiation (high-pressure mercury lamps 300 mW/$cm^2$, 1,000 mJ/$cm^2$) to obtain a laminate having a hard coat layer. The obtained laminate was cut out, and the surface tack resistance, elongation, pencil hardness, scratch resistance, bending resistance and sunscreen resistance of the hard coat layer were evaluated by the following methods, and the results are shown in Table 10. In Examples 57 and 60, the laminates after active energy ray curing were placed in a thermostatic chamber at 80°C and heat-treated for 8 hours, and then various properties were evaluated.

<Evaluation of Surface Tackiness Resistance>

Using the obtained laminate, the surface of the film was touched with a finger and the stickiness was evaluated.

◎: There was no stickiness.
○: Slight stickiness was observed, but no trace of finger remained on the surface.
△: It was sticky and leaved finger marks on the surface.
×: Stickiness was severe and finger sticked to the surface.

<Elongation Rate Evaluation>

The obtained laminate was cut into a lengthwise 50 mm and a widthwise 15 mm, fixed to a Tensilon universal tester RTA-100 (manufactured by Orientec Co., Ltd.) with a chuck-to-chuck spacing 25 mm, and pulled in one direction at a ratio of 250 mm/min in an oven set at a temperature of 150°C while visually observing the appearance. The elongation percentage was calculated and evaluated by the following method.

$$\text{Elongation rate (\%)} = (\text{specimen length after test}/25) \times 100\%$$

◎: Elongation rate was 200% or more.
○: Elongation rate was 150% more and less than 200%.
△: Elongation rate was 110% or more and less than 150%.
×: Elongation rate was less than 110%.

<Surface Hardness Evaluation>

Using a test piece of the laminate, a pencil was scratched by about 10 mm at an angle of 45° in accordance with JIS K 5600, and then a hardest pencil having no scratches on the surface of the laminate was used as a pencil hardness, and the surface hardness was evaluated as follows.

◎: Pencil hardness was 2H or more.
◦: Pencil hardness was HB to H.
△: Pencil hardness was 3B to B.
×: Pencil hardness was 4B or less.

<Scratch Resistance Evaluation>

The test piece of the laminate was reciprocated 10 times with a steel wool of #0000 under a load of 200 g, and the surface of the laminate was visually observed to evaluate the scratch resistance.

◎: No peeling or scratching of the film was observed.
◦: Slight fine scratch was observed in a part of the membrane.
△: Muscular injuries were noted throughout the membrane.
×: Delamination of the film occurred.

<Bending Resistance Evaluation>

The test piece of the laminate was bent at 180° so that the coating surface was on the outer side, a 1 kg weight was placed on the test piece, and the test piece was left to stand for 10 minutes, and the presence or absence of cracks on the surface of the laminate was visually observed to evaluate the bending resistance.

◎: No cracking was seen.
◦: Bent portion was partially whitened.
△: Partial crack was observed in the bent portion.
×: Cracks were seen in the folds.

<Sunscreen Resistance Evaluation>

A sunscreen agent Ultra Sheer DRY-TOUCH SUNSCREEN SPF100+ (manufactured by Johnson End Johnson Co., Ltd.) was coated on the coated surface of the test piece of the laminate to have a diameter of about 1 cm. After heated at 80°C for 6 hours, allowed to cool, washed away with a neutral detergent, and the state of the surface was observed to evaluate the sunscreen agent resistance as described below.

◎: No traces of sunscreen were seen.
◦: Transparent marks are slightly visible in the sunscreen-coated parts.
△: White marks remain in the portion where the sunscreen was applied, and the surface was swollen.
×: The portion coated with the sunscreen was sticky and the surface was peeled off.

As is clear from the results in Table 10, laminates having a decorative coating layer (decorative coating film) can be easily obtained by applying the active energy ray curable decorative coating agent of Examples to the surface of a general-purpose plastic substrates and curing the active energy ray curable decorative coating agents. The surface (coated surface) of the obtained laminates have tackiness resistance, high hardness, and excellent scratch resistance and sunscreen resistance. In addition, the elongation percentage and the bending resistance of the obtained laminates were good. In particular, after curing with active energy rays, the stress between the coating surface and the substrate was relaxed by further heat treatment, and in the case where the cyclic substituents of the (meth)acrylate (A), which is an essential constituent component of the decorative coating agent, has an unsaturated bond, the unsaturated bond progressed thermal polymerization in the heat treatment step, and the surface (coating surface) hardness, scratch

resistance, and sunscreen resistance were further improved. On the other hand, in the curable composition of Comparative Example, similar decorative performance was not confirmed. The decoration coating agent of the present invention is suitably used for various decoration molding, decoration processing, and decoration printing such as decoration films, decoration sheets, and decoration coatings.

<Preparation and Evaluation of Active Energy Ray curable Dental Material>

Examples 64-68 and Comparative Example 23 and 24

The active energy ray curable composition obtained in Table 2, the (meth)acrylate (A), the polymerizable compound (B), the photopolymerization initiator (C), and other components were weighed and uniformly mixed at room temperature to prepare active energy ray curable dental materials. The solubility or dispersibility of the dental material (when an insoluble inorganic filler, pigment or the like is blended) was visually observed, and the storage stability was evaluated, and the results are shown in Table 11. In addition, a dental material cured product was prepared by the following method using a dental material, and the curability of the dental material and the surface smoothness, hardness, and adhesive strength of the obtained dental material cured product were evaluated, and the results are shown in Table 11. In Examples 62 and 65, the cured product after curing with the active energy ray was placed in a thermostatic machine at 80°C, and after heat treatment for 8 hours, various physical properties were evaluated.

<Solubility (Dispersibility) Evaluation>

◎: The resulting composition was uniform and transparent.
○: The resulting composition was homogeneous and translucent.
△: The resulting composition was cloudy, and it was difficult to judge homogeneity.
×: The resulting composition was completely immiscible.

<Storage Stability Evaluation>

The obtained compositions of Examples and Comparative Examples were placed in a light-shielding screw tube, the cap was closed, and the tube was stored under two conditions of 40°C for 1 month and 80°C for 2 weeks. The dissolved or dispersed state of the compositions after storage were confirmed to evaluate storage stability.

O: There was no change in state after storage under both conditions of 1 month at 40°C and 2 weeks at 80°C.
△: State change after storage was confirmed under any one of conditions of 1 month at 40°C and 2 weeks at 80°C.
×: The state change after storage was confirmed in both of the two conditions of 1 month at 40°C and 2 weeks at 80°C.

<Curability Evaluation>

Using the obtained compositions of Examples and Comparative Examples, a mold (20 mm×20 mm×10 mm) made of polytetrafluoroethylene having a hole with a diameter of 6 mm at the center was filled with the composition, pressed with a polypropylene film, and irradiated for 30 seconds with a dental light irradiator (Tokuso Power light, manufactured by Tokuyama Dental Co., Ltd., light power density 700 mW/cm$^2$, light intensity on irradiated surface 640 to 650 mW/cm$^2$, light sources are halogen lamps, irradiation aperture 8 mm) in close contact with the polypropylene film. The polypropylene film was peeled off and the cured product was touched by hand to confirm stickiness and the presence or absence of an uncured component.

◎: There was no stickiness (complete curing).
◦: There was some stickiness, but no finger marks remained on the surface (approximately fully cured, no wiping of uncured ingredients was necessary).
△: There was stickiness, and finger marks remained on the surface (incomplete curing, necessitating wiping of the uncured ingredients).
×: Stickiness was severe, and finger sticked to the surface (a large number of uncured components remained and could not be used as a cured film).

<Surface Smoothness Evaluation>

The surface of the cured product obtained in the curability evaluation was visually observed to confirm the smoothness and glossiness, and the surface smoothness was evaluated.

◎: The surface was smooth and glossy.
◦: The surface was almost smooth with smooth haze or slight irregularities.
△: The entire surface was cloudy, and some irregularities or grains were observed.
×: The surface was entirely cloudy and covered with granules.

<Hardness Evaluation>

The surface of the cured product obtained in the curability evaluation was buffed and the Knoop hardness was measured with a microhardness meter manufactured by Matsumoto Seiki Co., Ltd. at a load of 10 g for 20 seconds. The measurement temperature was 23°C.

◎: Knoop hardness was 200 KHN or more (corresponding to permanent tooth enamel).
◦: Knoop hardness was 70 KHN or more and less than 200 KHN (corresponding to dentin).
△: Knoop hardness was less than 70 KHN.
×: No measurement could be made since it did not cure.

<Adhesive Strength (Dentin Adhesive Strength)>

The forehead of a bovine forehead was ground with #1000 water-resistant abrasive paper under pouring water, a flat bonding dentin surface was scraped off, and dried by blowing compressed air for 10 seconds, and a tape having a hole with a diameter of 3 mm was attached thereto to set an adherend surface. Thereafter, an adhesive test piece was prepared by a known method (see the method described in JP2010-208964A). The adhesion test piece was immersed in water at 37°C for 24 hours, and then the tensile adhesion strength was measured using an Instron universal tester (crosshead speed: 2 mm/min), and the adhesion strength to enamel and dentin of the compositions obtained in Examples and Comparative Examples was determined. The value of the tensile adhesion strength was an average value of five test pieces.

◎: Adhesion strength between enamel and dentin was 20 Mpa or more.
◦: Adhesion strength between the enamel and the dentin was 20 MPa or more.
△: Adhesion strength between enamel and dentin was 7 Mpa or more.
×: Adhesion strength between enamel and dentin was less than 7 Mpa.

As is clear from the results in Table 11, the active energy ray curable dental materials of Examples have high solubility (or dispersibility), curability and storage stability, and the cured products obtained by curing them have good hardness, surface smoothness and adhesive strength. On the other hand, since the compositions of Comparative Examples had low solubility, curability, and storage stability were not sufficiently cured, the hardness and surface smoothness of the obtained cured product were low, and the adhesive strength was insufficient.

As shown in the evaluation results of these Examples and Comparative Examples, the active energy ray curable compositions containing the (meth)acrylate (A) having a specific structure and the polymerizable compound (B) according to the present invention have good wettability and adhesion to various substrates while having high transparency and good curability, and cured products obtained by curing them are excellent in water resistance, heat resistance, impact resistance, abrasion resistance and the like. Due to the interaction between the (meth)acrylate (A) and the polymerizable compound (B), the curable compositions exhibited low cure shrinkage when cured, and the obtained cured product had no internal stresses or strains, and thus had good durability. On the other hand, it is clear that a curable composition containing no (meth)acrylate (A) or polymerizable compound (B) and a cured product thereof do not have the various properties described above. That is, it was confirmed that various properties of the active energy ray curable composition of the present invention and the cured product obtained therefrom were due to the interaction between the (meth)acrylate (A) contained therein and the polymerizable compound (B).

[Table 1]

| (Meth)-acryl rate (A) | (Meth)-acryl rate group | Firstlinking group | Amide group | Second linking group | Cyclic substituents |
|---|---|---|---|---|---|
| A-1 | Acrylate | -(CH2)2- | Secondary amide | None | 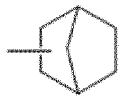 |
| A-2 | Acrylate | -(CH2)2- | Secondary amide | None | 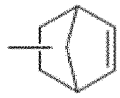 |
| A-3 | Acrylate | -(CH2)2- | Secondary amide | None | 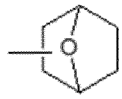 |
| A-4 | Meth-a crylate | -(CH2)2- | Secondary amide | None | 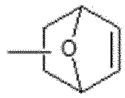 |
| A-5 | Acrylate | -(CH2)2- | Secondary amide | None | 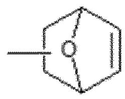 |
| A-6 | Acrylate | -(CH2)2- | Secondary amide | None | 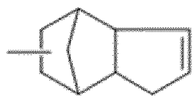 |
| A-7 | Acrylate | -(CH2)2- | Secondary amide | None | 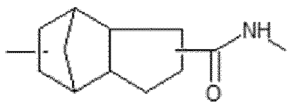 |
| A-8 | Meth-a crylate | -(CH2)2- | Methyl tertiary amide | None | 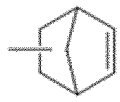 |
| A-9 | Acrylate | -(CH2)2- | Secondary amides | None | 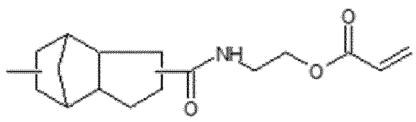 |
| A-10 | Acrylate | -(CH2)2- | Secondary amide | None | 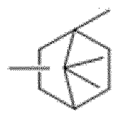 |
| A-11 | Acrylate | -(CH2)2- | Secondary amide | None | 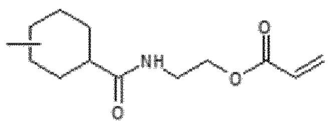 |
| A-12 | Acrylate | -CH2CH (CH3)- | Secondary amide | None | 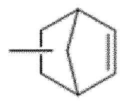 |

(continued)

| (Meth)-acryl rate (A) | (Meth)-acryl rate group | First linking group | Amide group | Second linking group | Cyclic substituents |
|---|---|---|---|---|---|
| A-13 | Acrylate | -(CH2)4- | Secondary amide | None | 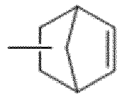 |
| A-14 | Acrylate | -(CH2)2- | Secondary amide | -(CH2)2- | 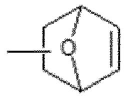 |
| A-15 | Acrylate | -(CH2)2- | Secondary amide | -(CH2)6- | 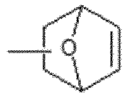 |

[Table 2]

| | | Active energy ray-curable composition | | | | | | | | Transparency | Curability (wavelength in nm) | | | Resistance cure shrinkage | Water resistance |
|---|---|---|---|---|---|---|---|---|---|---|---|---|---|---|---|
| | | (Meth) acrylate (A) | | Polymerizable compound (B) | | | | Others | | | | | | | |
| | | | | Linear (b1) | | Cyclic (b2) | | | | | | | | | |
| | | Type | Mass (%) | Type | Mass (%) | Type | Mass (%) | Type | Mass (%) | | 365 | 385 | 405 | | |
| Examples | 1 | A-1 | 15 | b1-4<br>b1-10 | 20<br>40 | b2-6<br>b2-13 | 15<br>9 | C-2 | 1 | ◎ | ◎ | ◎ | ◎ | ◎ | ○ |
| | 2 | A-2 | 30 | b1-1<br>b1-11 | 40<br>27 | - | - | C-1 | 3 | ◎ | ◎ | ◎ | ◎ | ◎ | ○ |
| | 3 | A-3<br>A-15 | 20<br>10 | b1-13 | 30 | b2-2<br>b2-10 | 20<br>18 | C-3 | 2 | ◎ | ○ | ○ | ○ | ◎ | ○ |
| | 4 | A-4 | 40 | b1-5<br>b1-7 | 2<br>18 | b2-11 | 30 | C-1 | 10 | ○ | ◎ | ◎ | ◎ | ◎ | ○ |
| | 5 | A-5<br>A-14 | 20<br>30 | b1-6<br>b1-9 | 10<br>10 | b2-4<br>b2-7 | 5<br>20 | C-2 | 5 | ◎ | ◎ | ◎ | ◎ | ◎ | ◎ |
| | 6 | A-6 | 60 | b1-4<br>b1-12 | 5<br>5 | b2-5<br>b2-10 | 4<br>25 | C-1 | 1 | ◎ | ◎ | ◎ | ◎ | ◎ | ◎ |
| | 7 | A-7 | 70 | b1-3 | 1 | b2-9 | 28 | C-2 | 1 | ○ | ◎ | ◎ | ◎ | ◎ | ◎ |
| | 8 | A-8 | 10 | b1-6<br>b1-10 | 5<br>13 | b2-8<br>b2-11 | 50<br>20 | C-1 | 2 | ○ | ◎ | ◎ | ○ | ○ | ◎ |
| | 9 | A-9 | 0.5 | b1-8<br>b1-12 | 40<br>34 | b2-3<br>b2-12 | 10<br>10 | C-3<br>D-2 | 5<br>0.5 | ○ | ◎ | ○ | ○ | ○ | ○ |
| | 10 | A-10 | 80 | b1-2 | 15 | b2-9 | 0.5 | C-2 | 4.5 | ○ | ◎ | ◎ | ◎ | ◎ | ◎ |
| | 11(*) | A-2<br>A-6 | 75<br>24.4 | b1-2 | 0.5 | - | - | C-3 | 0.1 | ○ | ○ | ○ | △ | ◎ | ○ |
| | 12 | A-1 | 5 | b1-12 | 80 | b2-2 | 12 | C-2 | 3 | ○ | ◎ | ◎ | ◎ | ○ | ○ |
| | 13 | A-2 | 65 | b1-7<br>b1-13 | 5<br>20 | b2-7 | 5 | C-3 | 5 | ◎ | ◎ | ◎ | ◎ | ◎ | ◎ |
| | 14 | A-11 | 45 | b1-10 | 40 | b2-2 | 10 | C-1 | 5 | ◎ | ◎ | ◎ | ◎ | ◎ | ◎ |
| | 15 | A-12 | 0.1 | b1-2<br>b1-7 | 35<br>20 | b2-6<br>b2-12 | 25<br>15 | C-3 | 4.9 | ◎ | ○ | ○ | ○ | △ | ○ |
| | 16 | A-13 | 25 | - | - | b2-2<br>b2-11 | 50<br>20 | C-2 | 5 | ○ | ◎ | ◎ | ◎ | ○ | ○ |
| Comparative Examples | 1 | A-1 | 50 | - | - | - | - | b3-1<br>C-1 | 45<br>5 | △ | △ | × | × | △ | × |
| | 2 | - | - | b1-8 | 60 | b2-7<br>b2-10 | 30<br>5 | C-3 | 5 | △ | △ | △ | × | × | × |
| | 3 | - | - | b1-4<br>b1-11 | 40<br>20 | b2-7 | 35 | C-2 | 5 | △ | △ | △ | × | × | × |
| | 4(*) | A-7 | 100 | - | - | - | - | - | - | ○ | △ | × | × | △ | × |

(*) In Example 11 and Comparative Example 4, curable compositions were prepared using ethyl acetate (50% by mass) as a solvent, and the transparency was evaluated. In other evaluations, after coating, the coated film was dried at 80 °C for 2 minutes to remove the solvent, and then irradiated with ultraviolet rays for evaluation. The density before curing in the curing shrinkage evaluation is a value measured before adding a solvent.

[Table 3]

| | | Ink composition | | | | Viscosity | Pigment dispersibility | Curability | Surface drying property | Ejection stablity | Sharpness |
|---|---|---|---|---|---|---|---|---|---|---|---|
| | | Examples etc curable composition | | Others | | | | | | | |
| | | Type | Mass (%) | Type | Mass (%) | | | | | | |
| Examples | 17 | Example 2 | 60 | b2-7 | 30 | ◎ | ◎ | ◎ | ◎ | ◎ | ◎ |
| | | | | C-3 | 5 | | | | | | |
| | | | | D-5 | 5 | | | | | | |
| | 18 | Example 1 | 40 | b1-1 | 20 | ◎ | - | ◎ | ◎ | ◎ | - |
| | | | | b1-13 | 15 | | | | | | |
| | | | | b2-2 | 20 | | | | | | |
| | | | | C-1 | 5 | | | | | | |
| | 19 | Example 12 | 30 | - | - | ○ | - | ◎ | ◎ | ○ | - |
| | | Example 14 | 70 | | | | | | | | |
| | 20 | - | - | A-8 | 20 | ◎ | ◎ | ◎ | ◎ | ◎ | ◎ |
| | | | | b1-2 | 50 | | | | | | |
| | | | | b1-11 | 25 | | | | | | |
| | | | | D-5 | 5 | | | | | | |
| | 21 | Example 10 | 10 | b1-10 | 20 | ◎ | ◎ | ◎ | ◎ | ◎ | ◎ |
| | | | | b2-2 | 60 | | | | | | |
| | | | | C-2 | 5 | | | | | | |
| | | | | D-5 | 5 | | | | | | |
| | 22 | - | - | A-2 | 25 | ◎ | ◎ | ◎ | ◎ | ◎ | ◎ |
| | | | | b1-1 | 20 | | | | | | |
| | | | | b1-12 | 25 | | | | | | |
| | | | | b2-7 | 20 | | | | | | |
| | | | | C-1 | 5 | | | | | | |
| | | | | D-5 | 5 | | | | | | |
| Comparative Exampls | 5 | Comparative Example 1 | 90 | C-1 | 5 | △ | × | △ | × | × | × |
| | | | | D-5 | 5 | | | | | | |
| | 6 | Comparative Example 2 | 85 | C-2 | 5 | △ | × | △ | × | × | × |
| | | | | D-1 | 5 | | | | | | |
| | | | | D-5 | 5 | | | | | | |
| | 7 | Comparative Example 4 | 50 | b3-2 | 20 | × | - | × | × | × | × |
| | | | | b3-3 | 20 | | | | | | |
| | | | | C-3 | 5 | | | | | | |
| | | | | D-1 | 5 | | | | | | |
| | 8 | | | A-10 | 30 | × | × | △ | × | × | × |
| | | | | b3-3 | 20 | | | | | | |
| | | | | b3-4 | 40 | | | | | | |
| | | | | C-2 | 5 | | | | | | |
| | | | | D-5 | 5 | | | | | | |

[Table 4]

| | | Ink composition for three-dimensional modeling | | | | Heat resistance | Impact resistance | Water resistance | Strength | Shaping accuracy |
|---|---|---|---|---|---|---|---|---|---|---|
| | | Examples etc curable composition or (A) | | Others | | | | | | |
| | | Type | Mass (%) | Type | Mass (%) | | | | | |
| Examples | 23 | Example 2 | 50 | b2-2 | 35 | ○ | ◎ | ◎ | ◎ | ◎ |
| | | | | b2-13 | 10 | | | | | |
| | | | | C-5 | 5 | | | | | |
| | 24 | Example 2 | 40 | b2-6 | 5 | ○ | ◎ | ◎ | ◎ | ○ |
| | | Example 14 | 30 | b2-10 | 25 | | | | | |
| | 25 | Example 7 | 50 | b1-12 | 30 | ○ | ◎ | ◎ | ◎ | ◎ |
| | | | | b2-2 | 20 | | | | | |
| | 26 | Example 8 | 100 | - | - | ○ | ◎ | ○ | ◎ | ◎ |
| | 27 | Example 4 | 20 | b1-10 | 40 | ○ | ◎ | ◎ | ◎ | ◎ |
| | | A-2 | 20 | b2-6 | 20 | | | | | |
| | 28 | A-2 | 5 | b1-2 | 35 | ○ | ◎ | ○ | ◎ | ◎ |
| | | | | b1-11 | 40 | | | | | |
| | | | | b2-3 | 10 | | | | | |
| | | | | b2-12 | 5 | | | | | |
| | | | | C-3 | 5 | | | | | |
| | 29 | A-1 | 20 | b1-6 | 10 | ○ | ◎ | ◎ | ○ | ◎ |
| | | | | b1-13 | 25 | | | | | |
| | | A-9 | 20 | b2-7 | 20 | | | | | |
| | | | | C-2 | 5 | | | | | |
| Comparative Examples | 9 | Comparative Example 1 | 40 | b3-1 | 20 | × | × | △ | × | × |
| | | | | b3-3 | 30 | | | | | |
| | | | | C-3 | 10 | | | | | |
| | 10 | Comparative Example 2 | 100 | - | - | × | × | × | × | △ |

[Table 5]

| | | Pressure-sensitive adhesive composition | | | | Curability | Adhesion | | | | Adhesive strength | | | Transparency | Stain resistance | Yellow discoloration resistance | Durability |
|---|---|---|---|---|---|---|---|---|---|---|---|---|---|---|---|---|---|
| | | Examples etc curable composition or (A) | | Others | | | PET | PMMA | ABS | Cu | PET | PC | ABS | | | | |
| | | Type | Mass (%) | Type | Mass (%) | | | | | | | | | | | | |
| Examples | 30 | Example 2 | 60 | b1-7 | 30 | ○ | ◎ | ◎ | ○ | ○ | ◎ | ◎ | ◎ | ◎ | ◎ | ◎ | ◎ |
| | | | | b2-12 | 10 | | | | | | | | | | | | |
| | 31 | Example 7 | 40 | b1-2 | 30 | ○ | ◎ | ○ | ◎ | ○ | ◎ | ○ | ◎ | ◎ | ◎ | ◎ | ◎ |
| | | | | b2-5 | 5 | | | | | | | | | | | | |
| | | | | b2-13 | 20 | | | | | | | | | | | | |
| | | | | C-2 | 5 | | | | | | | | | | | | |
| | 32 | Example 6 | 80 | b2-12 | 9.5 | ○ | ◎ | ○ | ○ | ○ | ◎ | ◎ | ◎ | ◎ | ◎ | ◎ | ◎ |
| | | Example 2 | 10 | D-6 | 0.5 | | | | | | | | | | | | |
| | 33 | Example 12 | 20 | b1-1 | 25 | ○ | ◎ | ◎ | ◎ | ◎ | ○ | ○ | ○ | ◎ | ◎ | ○ | ◎ |
| | | | | b2-6 | 3 | | | | | | | | | | | | |
| | | A-12 | 20 | b2-10 | 30 | | | | | | | | | | | | |
| | | | | C-1 | 2 | | | | | | | | | | | | |
| | 34 | A-2 | 5 | b1-8 | 60 | ○ | ○ | ◎ | ○ | ◎ | ○ | ◎ | ◎ | ◎ | ○ | ◎ | ◎ |
| | | | | b2-3 | 10 | | | | | | | | | | | | |
| | | | | b2-9 | 15 | | | | | | | | | | | | |
| | | | | b2-12 | 5 | | | | | | | | | | | | |
| | | | | C-3 | 5 | | | | | | | | | | | | |
| | 35 | A-10 | 5 | b1-9 | 8 | ◎ | ◎ | ◎ | ○ | ○ | ◎ | ◎ | ◎ | ◎ | ◎ | ◎ | ◎ |
| | | | | b2-8 | 25 | | | | | | | | | | | | |
| | | A-13 | 5 | b2-12 | 50 | | | | | | | | | | | | |
| | | | | C-2 | 5 | | | | | | | | | | | | |
| | | | | D-6 | 2 | | | | | | | | | | | | |
| Comparative Examples | 11 | Comparative Example 1 | 80 | b3-3 | 15 | △ | × | △ | × | × | × | △ | × | △ | × | × | × |
| | | | | C-2 | 5 | | | | | | | | | | | | |
| | 12 | Comparative Example 2 | 70 | b1-7 | 15 | △ | △ | × | × | × | △ | × | × | × | × | △ | × |
| | | | | b2-11 | 10 | | | | | | | | | | | | |
| | | | | C-2 | 5 | | | | | | | | | | | | |

[Table 6]

| | | Adhesive composition | | | | Curing Method | Substrate/ substrate | Adhesive strength | Impact resistance |
|---|---|---|---|---|---|---|---|---|---|
| | | Examples etc curable composition or | | Others | | | | | |
| | | Type | Mass (%) | Type | Mass (%) | | | | |
| Examples | 36 | Example 2 | 50 | b1-11 | 20 | UV | PET/PMMA | ◎ | ◎ |
| | | | | b2-3 | 15 | | | | |
| | | | | b2-11 | 10 | | | | |
| | | | | C-3 | 5 | | | | |
| | 37 | A-1 | 5 | b1-3 | 5 | UV | PET/PET | ◎ | ○ |
| | | | | b2-2 | 50 | | | | |
| | | | | b2-10 | 30 | | | | |
| | | | | b2-13 | 2 | | | | |
| | | | | C-2 | 8 | | | | |
| | 38 | Example 13 | 70 | b1-4 | 6 | UV Heat | PET/SPCC | ◎ | ◎ |
| | | | | b2-4 | 5 | | | | |
| | | A-6 | 12 | C-3 | 5 | | | | |
| | | | | D-6 | 2 | | | | |
| | 39 | A-2 | 30 | b1-2 | 40 | UV Heat | PC/Cu | ◎ | ◎ |
| | | | | b1-12 | 10 | | | | |
| | | | | b2-9 | 10 | | | | |
| | | | | C-1 | 10 | | | | |
| | 40 | Example 16 | 95 | b1-6 | 5 | UV | PC/PC | ◎ | ◎ |
| | 41 | Example 10 | 10 | b1-1 | 20 | UV | PET/PP | ○ | ◎ |
| | | | | b1-10 | 45 | | | | |
| | | Example 14 | 20 | b2-5 | 5 | | | | |
| Comparative Examples | 13 | A-6 | 50 | b3-3 | 35 | UV Heat | PET/PET | △ | × |
| | | | | C-3 | 10 | | | | |
| | | | | D-6 | 5 | | | | |
| | 14 | Comparative Example 3 | 90 | b1-8 | 10 | UV | PC/PC | × | × |

[Table 7]

| | | Coating composition | | | | Wettability | | | | | | | | | | | Curability (curing method) | Appearance of coating film | Abrasion Resistance |
|---|---|---|---|---|---|---|---|---|---|---|---|---|---|---|---|---|---|---|---|
| | | Examples etc curable composition or (A) | | Others | | | | | | | | | | | | | | | |
| | | Type | Mass (%) | Type | Mass (%) | PET | PMMA | PC | PO | PVC | ABS | SPCC | SUS | Al | Cu | HDF | | | |
| Examples | 42 | Examplet 2 | 90 | b2-12 | 10 | ◎ | ◎ | ◎ | ◎ | ◎ | ◎ | ◎ | ◎ | ◎ | ◎ | ◎ | ○ (EB after UV) | ◎ | ○ |
| | 43 | Example 5 | 70 | b1-12<br>C-1<br>D-2<br>D-5 | 20<br>5<br>2<br>3 | ◎ | ◎ | ◎ | ◎ | ◎ | ◎ | ◎ | ◎ | ○ | ○ | ◎ | ○ (UV) | ◎ | ○ |
| | 44 | Example 3 | 10 | b1-13<br>b2-11<br>C-2<br>D-3 | 40<br>40<br>5<br>5 | ◎ | ◎ | ◎ | ○ | ◎ | ◎ | ◎ | ◎ | ◎ | ◎ | ◎ | ○ (UV) | ◎ | ○ |
| | 45 | Example 8 | 99.9 | D-4 | 0.1 | ◎ | ◎ | ◎ | ◎ | ◎ | ◎ | ◎ | ◎ | ○ | ◎ | ◎ | ○ (EB) | ◎ | ○ |
| | 46 | A-2 | 60 | b1-10<br>b2-2<br>b2-12<br>C-3 | 10<br>10<br>7<br>3 | ◎ | ◎ | ◎ | ◎ | ◎ | ◎ | ◎ | ◎ | ◎ | ◎ | ◎ | ○ (EB after UV) | ○ | ○ |
| | 47 | A-1<br>A-9 | 15<br>25 | b1-13<br>b2-11<br>C-2<br>D-4 | 34<br>20<br>5<br>1 | ◎ | ◎ | ◎ | ◎ | ◎ | ◎ | ◎ | ◎ | ◎ | ◎ | ◎ | ○ (UV) | ◎ | ○ |
| Comparative Examples | 15 | Comparative Example 1 | 95 | D-2 | 5 | △ | × | × | × | △ | △ | △ | × | × | △ | △ | △ (UV) | △ | × |
| | 16 | Comparative Example 2 | 80 | b2-10<br>C-3 | 15<br>5 | △ | × | × | × | △ | △ | △ | △ | × | × | △ | △ (UV) | △ | × |

[Table 8]

| | | Sealing Composition | | | | Curing method | Transparency | Water resistance | Outgassing resistance | Heat cycle resistance | Yellowing resistance to heat and humidity | Corrosion resistance |
|---|---|---|---|---|---|---|---|---|---|---|---|---|
| | | Examples etc curable composition or (A) | | Others | | | | | | | | |
| | | Type | Mass (%) | Type | Mass (%) | | | | | | | |
| Examples | 48 | Example 1 | 50 | b1-8 | 35 | UV | ◎ | ○ | ◎ | ◎ | ◎ | ◎ |
| | | | | b2-13 | 10 | | | | | | | |
| | | | | C-2 | 5 | | | | | | | |
| | 49 | Example 4 | 20 | - | - | UV | ◎ | ◎ | ◎ | ◎ | ◎ | ◎ |
| | | Example 9 | 80 | | | | | | | | | |
| | 50 | Example 16 | 40 | b1-2 | 15 | UV | ○ | ◎ | ◎ | ◎ | ◎ | ◎ |
| | | A-2 | 10 | b1-10 | 20 | | | | | | | |
| | | | | C-3 | 5 | | | | | | | |
| | 51 | Example 14 | 95 | b2-13 | 3 | UV | ◎ | ○ | ◎ | ◎ | ◎ | ◎ |
| | | | | D-2 | 2 | | | | | | | |
| | 52 | A-12 | 50 | b2-8 | 10 | EB | ○ | ◎ | ◎ | ◎ | ○ | ◎ |
| | | | | b2-12 | 40 | | | | | | | |
| Comparative Examples | 17 | Comparative Example 1 | 100 | - | - | UV | △ | × | × | × | × | × |
| | 18 | Comparative Example 3 | 70 | b1-9 | 20 | UV | △ | × | △ | × | △ | × |
| | | | | C-3 | 3 | | | | | | | |
| | | | | D-1 | 7 | | | | | | | |

[Table 9]

| | | Nail Cosmetic Composition | | | | Curability | Adhesion | Surface Hardness | Surface Glossiness |
|---|---|---|---|---|---|---|---|---|---|
| | | Examples etc curable composition or (A) | | Others | | | | | |
| | | Type | Mass (%) | Type | Mass (%) | | | | |
| Examples | 53 | Example 2 | 90 | b2-12 | 7 | ◎ | ○ | ○ | ○ |
| | | | | C-2 | 3 | | | | |
| | 54 | Example 1 | 20 | b1-2 | 20 | ◎ | ◎ | ○ | ○ |
| | | | | b1-13 | 30 | | | | |
| | | | | b2-7 | 25 | | | | |
| | | | | C-3 | 5 | | | | |
| | 55 | Example 6 | 15 | b1-3 | 20 | ◎ | ◎ | ○ | ○ |
| | | | | b2-8 | 20 | | | | |
| | | Example 7 | 10 | b2-12 | 30 | | | | |
| | | | | C-1 | 5 | | | | |
| | 56 | Example 8 | 70 | b2-3 | 5 | ○ | ◎ | ○ | ○ |
| | | | | b2-10 | 15 | | | | |
| | | A-12 | 5 | C-2 | 5 | | | | |
| | 57 | A-3 | 20 | b1-1 | 40 | ◎ | ◎ | ○ | ○ |
| | | | | b1-13 | 20 | | | | |
| | | A-9 | 5 | b2-6 | 10 | | | | |
| | | | | C-1 | 5 | | | | |
| | 58 | A-10 | 15 | b1-6 | 10 | ◎ | ◎ | ○ | ○ |
| | | | | b2-2 | 50 | | | | |
| | | | | b2-13 | 20 | | | | |
| | | | | C-3 | 5 | | | | |
| Comparative Examples | 19 | Comparative Example 1 | 90 | b3-4 | 5 | △ | × | × | × |
| | | | | C-3 | 5 | | | | |
| | 20 | Comparative Example 2 | 50 | b1-12 | 30 | △ | × | △ | × |
| | | | | b2-11 | 15 | | | | |
| | | | | C-2 | 5 | | | | |

[Table 10]

| | | Decorative coating agent | | | | Curing method | Surface tackiness resistance | Elongation rate | Surface hardness | Scratch resistance | Bending resistance | Sunscreen resistance |
|---|---|---|---|---|---|---|---|---|---|---|---|---|
| | | Examples etc curable composition or (A) | | Others | | | | | | | | |
| | | Type | Mass (%) | Type | Mass (%) | | | | | | | |
| Examples | 59 | Example 2 | 70 | b2-2 | 17 | UV Heating | ◎ | ◎ | ◎ | ◎ | ◎ | ◎ |
| | | | | b2-13 | 10 | | | | | | | |
| | | | | C-3 | 3 | | | | | | | |
| | 60 | A-9 | 5 | b1-3 | 5 | UV | ◎ | ○ | ◎ | ◎ | ◎ | ○ |
| | | | | b1-12 | 40 | | | | | | | |
| | | | | b2-7 | 40 | | | | | | | |
| | | | | b2-11 | 5 | | | | | | | |
| | | | | C-1 | 5 | | | | | | | |
| | 61 | Example 6 | 60 | b1-1 | 10 | UV | ◎ | ◎ | ◎ | ◎ | ○ | ◎ |
| | | A-15 | 10 | b1-13 | 10 | | | | | | | |
| | | | | C-2 | 10 | | | | | | | |
| | 62 | Example 3 | 30 | b1-9 | 2 | UV Heating | ◎ | ○ | ◎ | ◎ | ◎ | ◎ |
| | | Example 13 | 40 | b2-6 | 25 | | | | | | | |
| | | | | b2-12 | 3 | | | | | | | |
| | 63 | A-1 | 5 | b1-11 | 30 | UV | ◎ | ◎ | ◎ | ◎ | ◎ | ◎ |
| | | | | b2-2 | 30 | | | | | | | |
| | | A-2 | 10 | b2-13 | 20 | | | | | | | |
| | | | | C-1 | 5 | | | | | | | |
| Comparative Example | 21 | Comparative Example 1 | 60 | b3-3 | 25 | UV | △ | × | △ | × | × | × |
| | | | | C-3 | 5 | | | | | | | |
| | | | | D-1 | 10 | | | | | | | |
| | 22 | Comparative Example 3 | 100 | | | UV | △ | × | × | △ | × | × |

[Table 11]

| | | Dental composition | | | | Curing method | Solubility or Dispersibility | Curability | Hardness | Surface Smoothness | Adhesion Strength | Storage Stability |
|---|---|---|---|---|---|---|---|---|---|---|---|---|
| | | Examples etc curable composition or (A) | | Others | | | | | | | | |
| | | Type | Mass (%) | Type | Mass (%) | | | | | | | |
| Examples | 64 | Example 2 | 70 | b1-13<br>b2-7 | 20<br>10 | UV<br>Heat | ◎ | ◎ | ◎ | ○ | ◎ | ○ |
| | 65 | Example 1<br>Example 7 | 30<br>20 | b1-2<br>b2-9<br>C-2<br>D-7 | 20<br>20<br>5<br>5 | UV | ○ | ◎ | ◎ | ◎ | ◎ | ○ |
| | 66 | Example 14 | 40 | b1-6<br>b2-2<br>b2-7<br>b2-11<br>C-3 | 10<br>20<br>20<br>7<br>3 | UV | ◎ | ◎ | ◎ | ◎ | ◎ | ○ |
| | 67 | Example 15<br>A-2 | 80<br>1 | b1-12<br>D-7 | 15<br>4 | UV<br>Heating | ◎ | ◎ | ◎ | ◎ | ○ | ○ |
| | 68 | A-10 | 20 | b1-2<br>b1-12<br>b2-10<br>C-3 | 30<br>35<br>10<br>5 | UV | ◎ | ◎ | ○ | ◎ | ◎ | ○ |
| Comparative Examples | 23 | Comparative Example 2 | 100 | | | UV | △ | △ | × | × | × | × |
| | 24 | Comparative Example 4 | 20 | b3-3<br>C-2<br>D-2<br>D-5 | 55<br>10<br>10<br>5 | UV | △ | × | × | × | △ | × |

INDUSTRIAL APPLICABILITY

As described above, the active energy ray curable composition of the present invention contains the specific (meth)acrylate (A) and the polymerizable compound (b1) having one or more chain substituents and/or the polymerizable compound (b2) having one or more cyclic substituents, whereby a cured product having high transparency and good curability, excellent wettability to various substrates having a wide range of polarities from low polarity to high polarity, low curing shrinkage, excellent water resistance, impact resistance, abrasion resistance, and durability can be obtained. The curable composition can be used for an active energy ray curable ink composition, an ink composition for two dimensional or three-dimensional modeling, a pressure-sensitive adhesive composition, an adhesive composition, a coating composition, a sealing composition, a nail cosmetic, a decorative coating agent, a dental material, and the like.

## Claims

**1.** An active energy ray curable composition comprising a (meth)acrylate (A) having one or more amide groups and one or more cyclic substitutions in the molecule, and a polymerizable compound (B) (excluding A) having one or more ethylenically unsaturated groups in the molecule, wherein the polymerizable compound (B) at least contains a polymerizable compound (b1) having one or more chain substituents having 1 to 36 carbon atoms and/or a polymerizable compound (b2) having one or more cyclic substituents having 3 to 20 carbon atoms.

**2.** The active energy ray curable composition according to claim 1, wherein the substituents of the (meth)acrylate (A) are one or more substituents selected from represents saturated or unsaturated multicyclic aliphatic rings, mono-or multicyclic aromatic rings, saturated or unsaturated aliphatic heterocycles, and aromatic heterocycles.

3. The active energy ray curable composition according to claims 1 or 2, wherein the (meth)acrylate (A) has an amide group between the (meth)acrylate and the cyclic substituents.

4. The active energy ray curable composition according to any one of claims 1 to 3, wherein the polymerizable compound (B) has one or more groups selected from (meth)acrylamide, (meth)acrylate, vinyl groups, vinyl ether, alkyl vinyl ether, allyl groups, (meth)allyl ether, and maleimide as the ethylenically unsaturated group.

5. The active energy ray curable composition according to any one of claims 1 to 4, wherein the content of (meth)acrylate (A) with respect to the entire active energy ray curable composition is 0. 1 to 99.5% by mass, the content of the polymerizable compound (b1) is 0.3 to 80% by mass, and the content of the polymerizable compound (b2) is 0.2 to 70% by mass.

6. The active energy ray curable composition according to any one of claims 1 to 5, which has a curing shrinkage rate of 5% or less.

7. The active energy ray curable composition according to any one of claims 1 to 6, wherein its cured product has a saturated water absorption rate of 10% or less.

8. An ink composition comprising the active energy ray curable composition according to any one of claims 1 to 7.

9. An ink composition for two-dimensional or three-dimensional modeling comprising the active energy ray curable composition according to any one of claims 1 to 7.

10. A pressure-sensitive adhesive composition comprising the active energy ray curable composition according to any one of claims 1 to 7.

11. An adhesive composition comprising the active energy ray curable composition according to any one of claims 1 to 7.

12. A coating composition comprising the active energy ray curable composition according to any one of claims 1 to 7.

13. A sealing composition comprising the active energy ray curable composition according to any one of claims 1 to 7.

14. A nail cosmetic comprising the active energy ray curable composition according to any one of claims 1 to 7.

15. A decorative coating agent comprising the active energy ray curable composition according to any one of claims 1 to 7.

16. A dental comprising the active energy ray curable composition according to any one of claims 1 to 7.

**INTERNATIONAL SEARCH REPORT**

International application No.
**PCT/JP2022/042300**

**A. CLASSIFICATION OF SUBJECT MATTER**

***C08F 220/36***(2006.01)i; ***C08F 290/06***(2006.01)i; ***C09D 4/02***(2006.01)i; ***C09D 5/00***(2006.01)i; ***C09D 11/101***(2014.01)i; ***C09D 11/30***(2014.01)i; ***C09J 4/02***(2006.01)i
FI: C08F220/36; C08F290/06; C09D4/02; C09D5/00 Z; C09D11/101; C09D11/30; C09J4/02

According to International Patent Classification (IPC) or to both national classification and IPC

**B. FIELDS SEARCHED**

Minimum documentation searched (classification system followed by classification symbols)

C08F220/36; C08F290/06; C09D4/02; C09D5/00; C09D11/101; C09D11/30; C09J4/02

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2018-95626 A (KJ CHEMICALS CORP) 21 June 2018 (2018-06-21) claims, paragraphs [0007], [0011]-[0021], [0025]-[0029], examples | 1-16 |
| A | WO 2015/122470 A1 (MITSUBISHI GAS CHEMICAL CO) 20 August 2015 (2015-08-20) entire text | 1-16 |
| A | WO 2019/163570 A1 (DAIKIN IND LTD) 29 August 2019 (2019-08-29) entire text | 1-16 |
| P, X | JP 2022-99528 A (KJ CHEMICALS CORP) 05 July 2022 (2022-07-05) entire text, in particular, paragraphs [0002], [0080], [0091], [0101], [0115], example 1 | 1-4, 6-13 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 January 2023** | **24 January 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/042300**

| Patent document cited in search report | Publication date (day/month/year) | Patent family member(s) | Publication date (day/month/year) |
|---|---|---|---|
| JP 2018-95626 A | 21 June 2018 | (Family: none) | |
| WO 2015/122470 A1 | 20 August 2015 | US 2016/0355461 A1<br>entire text<br>EP 3106454 A1<br>CN 105980347 A | |
| WO 2019/163570 A1 | 29 August 2019 | US 2020/0377774 A1<br>entire text<br>EP 3757187 A1<br>CN 111742031 A | |
| JP 2022-99528 A | 05 July 2022 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2021123720 A
- JP 2018095626 A
- JP 2010208964 A